# EUROPEAN PATENT APPLICATION

(11) **EP 4 299 589 A1**
(43) Date of publication of application: **03.01.2024**
(21) Application number: 21927468.5
(22) Date of filing: 16.07.2021
(51) Int. Cl.: C07K 16/28, C07K 19/00, C12N 15/13, C12N 5/10, A61K 39/395, A61K 39/00, A61K 47/68, A61P 35/00, A61P 35/02, G01N 33/68

(54) **ANTI-HUMAN CD73 ANTIBODY AND USE THEREOF**

(30) Priority: 24.02.2021 CN 202110202193; 18.03.2021 CN 202110292998
(71) Applicant: Novoprotein Scientififc Inc., Suzhou, Jiangsu 215200 (CN); Novoprotein Scientific (Shanghai) Inc., Shanghai 201203 (CN)
(72) Inventor: LI, Debin, Suzhou, Jiangsu 215200 (CN); SONG, Zuowei, Suzhou, Jiangsu 215200 (CN); LU, Na, Suzhou, Jiangsu 215200 (CN); YU, Futao, Shanghai 201203 (CN)
(74) Representative: Lavoix
(86) International application number: PCT/CN2021/106917
(87) International publication number: WO 2022/179039

(57) **Abstract**

Provided is a novel anti-human CD73 humanized antibody, which is capable of specifically binding to a CD73 antigen and has high affinity and biological activity.

## Description

### Technical field

The invention relates to the field of biomedicine, in particular to an anti-human CD73 antibody and use thereof.

### Background

The CD73, the differentiation cluster 73, also called 5' - nucleotidase, are anchored on the cell membrane by GPI (glycosyl phosphatidylinositol), and the structure is a dimer. The CD73 plays an important role in a tumor microenvironment, and the AMP can be converted into adenosine. The adenosine is combined with a specific G protein coupling receptor, so that the immune cells are infiltrated abnormally in the tumor site, the proliferation and migration of various cancers are regulated and controlled, the function of the immune system related to the T cells is mainly inhibited, and the immune system is a marker with poor tumor prognosis.

In a large number of studies in the past, it is indicated that inhibition of CD73 can prevent the occurrence and transfer of tumors. Whether a small molecule enzyme inhibitor or a macromolecular antibody of CD73 shows an anti-tumor effect. CD73 is expressed on multiple tumor cell surfaces, including lung cancer, melanoma, colon cancer, pancreatic cancer and other tumor cells. In addition, various immune cells are also affected by CD73, and CD73 is expressed on the Treg surface and participates in inhibitory regulation of Treg on immunoactivation; the secreted adenosine can be combined with an adenosine receptor on a DC cell, so that the function of DC is inhibited; and CD73 is highly expressed on M2 -type macrophages, so that the metastasis of tumors is enhanced.

The potential of CD73 target in tumor treatment is huge, and the antibody of CD73 can also enhance the effect of PD1 antibody and CTLA4 antibody. However, there is no CD73 specific targeting antibody to appear on the market at present. In addition, since the mouse monoclonal antibody can cause a human anti-mouse antibody response (human anti-mouse antibody, HAMA) during clinical treatment, it is limited in clinical treatment. Antibody humanization technology can greatly reduce the immunogenicity of mouse monoclonal antibodies.

Therefore, in view of the role and function of CD73 in various related diseases, the development anti-CD73humanized antibodies suitable for treating patients is needed in this field.

### Summary of the invention

The purpose of the present invention is to provide an anti-human CD73 antibody having high affinity and high bioactivity, and use thereof.

In the first aspect of the present invention, it provides a heavy chain variable region of anti-CD73 antibody, and the heavy variable region has the VH-CDR selected from the group consisting of:
(1) VH-CDR1 as shown in SEQ ID NO: 15 or 21,
(2) VH-CDR2 as shown in SEQ ID NO.: 16, and
(3) VH-CDR3 independently selected from the following group: SEQ ID NO.: 17, SEQ ID NO.: 22, SEQ ID NO.: 23, SEQ ID NO.: 24, SEQ ID NO.: 25, SEQ ID NO.: 26, SEQ ID NO.: 27.

In another preferred embodiment, the heavy chain variable region has the amino acid sequence as shown in SEQ ID NO.: 2.

In another preferred embodiment, the heavy chain variable region has the amino acid sequence as shown in SEQ ID NO.: 5.

In another preferred embodiment, the heavy chain variable region has the amino acid sequence as shown in SEQ ID NO.: 7.

In another preferred embodiment, the heavy chain variable region has the amino acid sequence as shown in SEQ ID NO.: 9.

In another preferred embodiment, the heavy chain variable region has the amino acid sequence as shown in SEQ ID NO.: 34.

In another preferred embodiment, the heavy chain variable region has the amino acid sequence as shown in SEQ ID NO.: 36.

In another preferred embodiment, the heavy chain variable region has the amino acid sequence as shown in SEQ ID NO: 37.

In another preferred embodiment, the heavy chain variable region has the amino acid sequence as shown in SEQ ID NO: 38.

In another preferred embodiment, the heavy chain variable region has the amino acid sequence as shown in SEQ ID NO: 39.

In another preferred embodiment, any one of the above amino acid sequences also includes a derivative sequence that is optionally with at least one amino acid added, deleted, modified, and/or substituted, and is capable of retaining the binding affinity of CD73.

In the second aspect of the present invention, it provides a heavy chain of an anti-CD73 antibody having the heavy chain variable region of the first aspect of the present invention.

In another preferred embodiment, the heavy chain of the antibody further includes heavy chain constant region.

In another preferred embodiment, the heavy chain constant region is of human origin, murine origin or rabbit origin, preferably human origin.

In another preferred embodiment, the heavy chain constant region is a human antibody heavy chain IgG1 constant region.

In the third aspect of the present invention, it provides a light chain variable region of anti-CD73 antibody, and the light chain variable region has the VL-CDR selected from the group consisting of:
(1) VL-CDR1 as shown in SEQ ID NO: 18,
(2) VL-CDR2 as shown in SEQ ID NO.: 19, and
(3) VL-CDR3 independently selected from the following group: SEQ ID NO.: 20, SEQ ID NO.: 28, SEQ ID NO.: 29, SEQ ID NO.: 30, SEQ ID NO.: 31, SEQ ID NO.: 32, SEQ ID NO.: 33.

In another preferred embodiment, the light chain variable region has the amino acid sequence as shown in SEQ ID NO.: 1.

In another preferred embodiment, the light chain variable region has the amino acid sequence as shown in SEQ ID NO.: 6.

In another preferred embodiment, the light chain variable region has the amino acid sequence as shown in SEQ ID NO.: 8.

In another preferred embodiment, the light chain variable region has the amino acid sequence as shown in SEQ ID NO.: 10.

In another preferred embodiment, the light chain variable region has the amino acid sequence as shown in SEQ ID NO.: 40.

In another preferred embodiment, the light chain variable region has the amino acid sequence as shown in SEQ ID NO.: 41.

In another preferred embodiment, the light chain variable region has the amino acid sequence shown in SEQ ID NO: 42.

In another preferred embodiment, the light chain variable region has the amino acid sequence shown in SEQ ID NO: 43.

In another preferred embodiment, the light chain variable region has the amino acid sequence shown in SEQ ID NO: 44.

In another preferred embodiment, the light chain variable region has the amino acid sequence shown in SEQ ID NO: 45.

In another preferred embodiment, any one of the above amino acid sequences also includes a derivative sequence that is optionally with at least one amino acid added, deleted, modified, and/or substituted, and is capable of retaining the binding affinity of CD73.

In the fourth aspect of the present invention, it provides a light chain of anti-CD73 antibody, wherein the light chain comprises the light chain variable region of the third aspect of the present invention.

In another preferred embodiment, the light chain of the antibody further includes a light chain constant region.

In another preferred embodiment, the heavy chain constant region is of human origin, murine origin or rabbit origin, preferably human origin.

In another preferred embodiment, the light chain constant region is a human antibody light chain kappa constant region.

In the fifth aspect of the present invention, it provides an anti-CD73 antibody, wherein the antibody comprises:
(1) the heavy chain variable region of the first aspect of the present invention; and/or
(2) the light chain variable region of the third aspect of the present invention;
   or the antibody comprises: the heavy chain of the second aspect of the present invention; and/or the light chain of the fourth aspect of the present invention,
   wherein, any one of the above amino acid sequences also includes a derivative sequence that is optionally with at least one amino acid added, deleted, modified, and/or substituted, and is capable of retaining the binding affinity of SEMA4D.

In another preferred embodiment, the number of added, deleted, modified and/or substituted amino acids is 1-5 (such as 1-3, preferably 1-2, more preferably 1).

In another preferred embodiment, the antibody is a humanized antibody.

In another preferred embodiment, the antibody is selected from the group consisting of an animal-derived antibody, a chimeric antibody, a humanized antibody, and a combination thereof.

In another preferred embodiment, the antibody is a double-chain antibody or a single-chain antibody.

In another preferred embodiment, the antibody is a monoclonal antibody.

In another preferred embodiment, the antibody is a monospecific, bispecific, or trispecific antibody.

In another preferred embodiment, the heavy chain variable region of the antibody further comprises a human-derived framework region, and/or the light chain variable region of the antibody further comprises a human-derived framework region.

In another preferred embodiment, the heavy chain variable region of the antibody further comprises a murine-derived framework region, and/or the light chain variable region of the antibody further comprises a murine-derived framework region.

In another preferred embodiment, the antibody comprises the heavy chain variable region of the first aspect of the present invention and the light chain variable region of the third aspect of the present invention;
wherein, the heavy chain variable region comprise CDRs selected from the following group:
   (1) VH-CDR1 as shown in SEQ ID NO: 15 or 21,
   (2) VH-CDR2 as shown in SEQ ID NO.: 16, and
   (3) VH-CDR3 independently selected from the following group: SEQ ID NO.: 17, SEQ ID NO.: 22, SEQ ID NO.: 23, SEQ ID NO.: 24, SEQ ID NO.: 25, SEQ ID NO.: 26 or SEQ ID NO.: 27; amd
the light chain variable region comprise CDRs selected from the following group:
   (1) VL-CDR1 as shown in SEQ ID NO: 18,
   (2) VL-CDR2 as shown in SEQ ID NO.: 19, and
   (3) VL-CDR3 independently selected from the following group: SEQ ID NO.: 20, SEQ ID NO.: 28, SEQ ID NO.: 29, SEQ ID NO.: 30, SEQ ID NO.: 31, SEQ ID NO.: 32 or SEQ ID NO.: 33.

In another preferred embodiment, the antibody comprises the heavy chain variable of the first aspect of the present invention and the light chain variable region of the third aspect of the present invention; wherein,
the three light chain CDRs of the light chain variable region and the three heavy chain CDRs of the heavy chain variable region are selected from the following group:
(Z1) VL-CDR1, VL-CDR2 and VL-CDR3 shown in SEQ ID NO: 18, 19, and 20; and VH-CDR1, VH-CDR2 and VH-CDR3 shown in SEQ ID NO: 15, 16, and 17;
(Z2) VL-CDR1, VL-CDR2 and VL-CDR3 shown in SEQ ID NO: 18, 19, and 20; and VH-CDR1, VH-CDR2 and VH-CDR3 shown in SEQ ID NO: 21, 16, and 17;
(Z3) VL-CDR1, VL-CDR2 and VL-CDR3 shown in SEQ ID NO: 18, 19, and 28; and VH-CDR1, VH-CDR2 and VH-CDR3 shown in SEQ ID NO: 15, 16, and 22;
(Z4) VL-CDR1, VL-CDR2 and VL-CDR3 shown in SEQ ID NO: 18, 19, and 29; and VH-CDR1, VH-CDR2 and VH-CDR3 shown in SEQ ID NO: 15, 16, and 23;
(Z5) VL-CDR1, VL-CDR2 and VL-CDR3 shown in SEQ ID NO: 18, 19, and 30; and VH-CDR1, VH-CDR2 and VH-CDR3 shown in SEQ ID NO: 15, 16, and 24;
(Z6) VL-CDR1, VL-CDR2 and VL-CDR3 shown in SEQ ID NO: 18, 19, and 31; and VH-CDR1, VH-CDR2 and VH-CDR3 shown in SEQ ID NO: 15, 16, and 25;
(Z7) VL-CDR1, VL-CDR2 and VL-CDR3 shown in SEQ ID NO: 18, 19, and 32; and VH-CDR1, VH-CDR2 and VH-CDR3 shown in SEQ ID NO: 15, 16, and 26; or
(Z8) VL-CDR1, VL-CDR2 and VL-CDR3 shown in SEQ ID NO: 18, 19, and 33; and VH-CDR1, VH-CDR2 and VH-CDR3 shown in SEQ ID NO: 15, 16, and 27.

In another preferred embodiment, the amino acid sequence of the light chain variable region (VL) of the anti-human CD73 antibody is as shown in SEQ ID NO: 1, 6, 8, 10, 40, 41, 42, 43, 44 or 45; and the amino acid sequence of the heavy chain variable region (VH) of the anti-human CD73 antibody is as shown in SEQ ID NO: 2, 5, 7, 9, 34, 35, 36, 37, 38 or 39.

In another preferred embodiment, the antibody is selected from the group consisting of:

| Antibody number | Clone Number | VH sequence number | VL sequence number |
|---|---|---|---|
| 1 | CQ137 | 2 | 1 |
| 2 | 137-1 | 5 | 6 |
| 3 | 137-2 | 7 | 8 |
| 4 | 137-3 | 9 | 10 |
| 5 | 137-4 | 34 | 40 |
| 6 | 137-5 | 35 | 41 |
| 7 | 137-6 | 36 | 42 |
| 8 | 137-7 | 37 | 43 |
| 9 | 137-8 | 38 | 44 |
| 10 | 137-9 | 39 | 45. |

In another preferred embodiment, the heavy chain variable region of the antibody comprises the amino acid sequence shown in SEQ ID NO.: 2, and the light chain variable region of the antibody comprises the amino acid sequence shown in SEQ ID NO.: 1.

In another preferred embodiment, the heavy chain variable region of the antibody comprises the amino acid sequence shown in SEQ ID NO.: 5, and the light chain variable region of the antibody comprises the amino acid sequence shown in SEQ ID NO.: 6.

In another preferred embodiment, the heavy chain variable region of the antibody comprises the amino acid sequence shown in SEQ ID NO.: 7, and the light chain variable region of the antibody comprises the amino acid sequence shown in SEQ ID NO.: 8.

In another preferred embodiment, the heavy chain variable region comprises the amino acid sequence shown in SEQ ID NO.: 9, and the light chain variable region comprises the amino acid sequence shown in SEQ ID NO.: 10.

In another preferred embodiment, the heavy chain variable region of the antibody comprises the amino acid sequence shown in SEQ ID NO.: 34, and the light chain variable region of the antibody comprises the amino acid sequence shown in SEQ ID NO.: 40.

In another preferred embodiment, the heavy chain variable region of the antibody comprises the amino acid sequence shown in SEQ ID NO.: 35, and the light chain variable region of the antibody comprises the amino acid sequence shown in SEQ ID NO.: 41.

In another preferred embodiment, the heavy chain variable region of the antibody comprises the amino acid sequence shown in SEQ ID NO.: 36, and the light chain variable region of the antibody comprises the amino acid sequence shown in SEQ ID NO.: 42.

In another preferred embodiment, the heavy chain variable region of the antibody comprises the amino acid sequence shown in SEQ ID NO.: 37, and the light chain variable region of the antibody comprises the amino acid sequence shown in SEQ ID NO.: 43.

In another preferred embodiment, the heavy chain variable region of the antibody comprises the amino acid sequence shown in SEQ ID N.: 38, and the light chain variable region of the antibody comprises the amino acid sequence shown in SEQ ID NO.: 44.

In another preferred embodiment, the heavy chain variable region of the antibody comprises the amino acid sequence shown in SEQ ID NO.: 39, and the light chain variable region of the antibody comprises the amino acid sequence shown in SEQ ID NO.: 45.

In another preferred embodiment, any one of the above amino acid sequences further includes a derivative sequence that is optionally added, deleted, modified and/or substituted with at least one (such as 1-3, preferably 1-2, more preferably 1) amino acid and can retain the affinity to bind to CD73.

In another preferred embodiment, the amino acid sequence of the heavy chain variable region has a sequence homology or sequence identity of at least 80%, 85%, 90%, 91%, 92%, 93%, 94%, 95%, 96%, 97%, 98% or 99% with the amino acid sequence shown in SEQ ID NO.: 2, 5, 7, 9, 34, 35, 36, 37, 38 or 39 in the sequence listing.

In another preferred embodiment, the amino acid sequence of the light chain variable region has a sequence homology or sequence identity of at least 80%, 85%, 90%, 91%, 92%, 93%, 94%, 95%, 96%, 97%, 98% or 99% with the amino acid sequence shown in SEQ ID NO.: 1, 6, 8, 10, 40, 41, 42, 43, 44 or 45 in the sequence listing.

In another preferred embodiment, the anti-CD73 antibody comprises light chain and heavy chain, and the light chain variable region of the light chain comprises the following three light chain CDRs:
VL-CDR1 as shown in SEQ ID NO.: 18,
VL-CDR2 as shown in SEQ ID NO.: 19, and
VL-CDR3 as shown in SEQ ID NO.: 20, 28, 29, 30, 31, 32 or 33; wherein, the heavy chain variable region of the heavy chain comprises the following three heavy chain CDRs:
   VH-CDR1 as shown in SEQ ID NO.: 15 or 21,
   VH-CDR2 as shown in SEQ ID NO.: 16, and
   VH-CDR3 as shown in SEQ ID NO.: 17, 22, 23, 24, 25, 26 or 27. In another preferred embodiment, wherein the light chain of the antibody comprises the three light chain CDRs and a light chain framework region for connecting the light chain CDR; and the heavy chain of the antibody comprises the three heavy chain CDRs and a heavy chain framework region for connecting the heavy chain CDR.

In another preferred embodiment, the antibody is a humanized antibody.

In another preferred embodiment, the antibody specifically binds to CD73.

In another preferred embodiment, the KD value (M) of the affinity of the antibody to human CD73 is 1.0E-10-2.0E-12.

In another preferred embodiment, the antibody is a double-chain antibody or a single-chain antibody.

In another preferred embodiment, the antibody is a monoclonal antibody.

In another preferred embodiment, the antibody is a monospecific, bispecific, or trispecific antibody.

In the sixth aspect of the present invention, it provides a recombinant protein having:
(i) the heavy chain variable region of the first aspect of the present invention, the heavy chain of the second aspect of the present invention, the light chain variable region of the third aspect of the present invention, the light chain of the fourth aspect of the present invention, or the antibody of the fifth aspect of the present invention; and (ii) optional tag sequence for assisting expression and/or purification.

In another preferred embodiment, the tag sequence comprises 6His tag.

In another preferred embodiment, the recombinant protein or polypeptide includes fusion protein.

In another preferred embodiment, the recombinant protein is a monomer, dimer, or polymer.

In another preferred embodiment, the recombinant protein further comprises an additional fusion element (or fusion polypeptide fragment) fused with the element (i).

In the seventh aspect of the present invention, it provides an antibody preparation, wherein the antibody preparation comprises:
(a) the antibody of the fifth aspect of the present invention, and
(b) a carrier, wherein the carrier comprises: a buffering agent, a sterile water, and an optional surfactant.

In the eighth aspect of the present invention, it provides a kit, wherein the kit contains the antibody preparation of the seventh aspect of the present invention, and a container containing the antibody preparation.

In the ninth aspect of the present invention, it provides a CARconstruct, wherein the scFv segment of the antigen binding region of the CAR construct specifically binds to binding region of CD73, and the scFv segment has the heavy chain variable region of the first aspect of the present invention and the light chain variable region of the third aspect of the present invention. In the tenth aspect of the present invention, it provides a recombinant immune cell, which expresses exogenous CAR construct of the ninth aspect of the present invention.

In another preferred embodiment, the immune cell is selected from the group consisting of an NK cell, a T cell.

In another preferred embodiment, the immune cells are derived from a human or non-human mammal, such as a mouse.

In the eleventh aspect of the invention, it provides an antibody drug conjugate comprising:
(a) an antibody moiety selected from the group consisting of the heavy chain variable region of the first aspect of the present invention, the heavy chain of the second aspect of the present invention, the light chain variable region of the third aspect of the present invention, the light chain of the fourth aspect of the present invention, or the antibody of the fifth aspect of the present invention, or the recombinant protein of the sixth aspect of the present invention, and a combination thereof; and (b) a coupling moiety coupled to the antibody moiety, which is selected from the group consisting of a detectable label, a drug, a toxin, a cytokine, a radionuclide, an enzyme, and a combination thereof.

In the twelfth aspect, it provides an active ingredient, wherein the active ingredient is selected from the group consisting of the antibody of the fifth aspect of the present invention, the recombinant protein of the sixth aspect of the present invention, the CAR construct of the ninth aspect of the present invention, the immune cell of the tenth aspect of the present invention, the antibody drug conjugate of the eleventh aspect of the present invention, and a combination thereof, wherein the active ingredient is used for:
(a) preparing a detection reagent or a kit;
(b) preparing a drug or preparation for preventing and/or treating CD73-related diseases; and/or
(c) preparing a drug or preparation for preventing and/or treating cancers or tumors associated with CD73-related diseases.

In another preferred embodiment, the cancer or tumor is selected from the group consisting of lung cancer, melanoma, colon cancer, pancreatic cancer, bladder cancer, breast cancer, ovarian cancer, prostate cancer, testicular cancer, esophageal cancer, gastrointestinal cancer, liver cancer, lymphoma, myeloma, leukemia.

In the thirteenth aspect of the present invention, it provides a pharmaceutical composition containing:
(i) an active ingredient, which is selected from the group consisting of the antibody of the fifth aspect of the present invention, the recombinant protein of the sixth aspect of the present invention, the CAR construct of the ninth aspect of the present invention, the immune cell of the tenth aspect of the present invention, the antibody drug conjugate of the eleventh aspect of the present invention, and a combination thereof, and
(ii) a pharmaceutically acceptable carrier.

In another preferred embodiment, the pharmaceutical composition is a liquid preparation.

In another preferred embodiment, the pharmaceutical composition is an injection.

In another preferred embodiment, the pharmaceutical composition is used for treating tumors.

In another preferred embodiment, the tumors are tumors that highly expressed CD73.

In the fourteenth aspect of the present invention, it provides a polynucleotide encoding a polypeptide selected from the group consisting of:
(1) the heavy chain variable region of the first aspect of the invention, the heavy chain of the second aspect of the invention, the light chain variable region of the third aspect of the invention, the light chain of the fourth aspect of the invention, or the antibody of the fifth aspect of the invention; or
(2) the recombinant protein of the sixth aspect of the present invention.
(3) the CAR construct of the ninth aspect of the present invention.

In the fifteenth aspect of the present invention, it provides a vector comprising a polynucleotide of the fourteenth aspect of the present invention.

In another preferred embodiment, the vector includes: a bacterial plasmid, a bacteriophage, a yeast plasmid, a plant cell virus, a mammalian cell virus such as an adenovirus, a retrovirus, or other vectors.

In the sixteenth aspect of the present invention, it provides a genetically engineered host cell, wherein the host cell comprises the vector of the fifteenth aspect of the present invention, or the genome thereof is integrated with the polynucleotide of the fourteenth aspect of the present invention.

In the seventeenth aspect of the present invention, it provides a method for *in vitro* non-diagnostic detection of CD73 protein in a sample, wherein the method comprises the steps:
*(1) in vitro* contacting the sample with the antibody of the fifth aspect of the present invention;
(2) detecting the formation of an antigen-antibody complex, wherein the formation of a complex indicates the presence of CD73 protein in the sample.

In the eighteenth aspect of the present invention, it provides a method for *in vitro* detection (including diagnostic or non-diagnostic) of CD73 protein in a sample, wherein the method comprises the steps:
*(1) in vitro* contacting the sample with the antibody of the fifth aspect of the present invention;
(2) detecting the formation of an antigen-antibody complex, wherein the formation of a complex indicates the presence of CD73 protein in the sample.

In the ninteenth aspect of the invention,it provides a method for treating CD73-related diseases, which comprises:
The antibody of the fifth aspect of the present invention, the drug conjugate of the antibody, or the CAR-T cell expressing the antibody, or a combination thereof, is administered to a subject in need thereof.

It should be understood that within the scope of the present invention, the various technical features of the present invention above and the various technical features specifically described hereinafter (as in the examples) may be combined with each other to constitute a new or preferred technical solution. And it needs not be described one by one, due to space limitations.

### DESCRIPTION OF THE DRAWINGS

FIG. 1 shows an affinity diagram of CQ137 and rhCD73 as determined by Biacore.
FIG. 2 shows an affinity detection diagram of CQ137 and recombinant monkey CD73 protein.
FIG. 3 shows an affinity detection diagram of CQ137 and SK-OV-3 cells.
FIG. 4 shows an affinity detection diagram of CQ137 and CHOK1-cynoCD73 cells.
FIG. 5 shows a comparison of rhCD73 inhibitory activity between two CD73 antibodies (CQ137 and MEDI-9447).
FIG. 6 shows a diagram of CQ137 inhibiting the activity of CD73 on SK-OV-3 cells.
FIG. 7 shows the activity map of the CQ137 inhibiting CD73 on CHOK1-cyno73 cells.
FIG. 8A shows the binding activity of humanized CD73 antibodies (137-1, 137-2, 137-3) and rhCD73.
FIG. 8B shows the binding activity of humanized CD73 antibody (137-1, 137-2, 137-3) and recombinant monkey CD73 protein.
FIG. 9 shows an affinity detection diagram of humanized CD73 antibodies (137-1, 137-2, 137-3) and SK-OV-3 cells.
FIG. 10 shows an affinity detection diagram of a humanized CD73 antibody (137-1, 137-2, 137-3) and CHOK1-cyno73 cells.
FIG. 11 shows the comparison of the inhibitory effect of humanized CD73 antibodies (137-1, 137-2, 137-3) on rhCD73 enzyme activity (RLU response multiple) , and RLU is a relative light unit.
FIG. 12 shows the comparison of humanized CD73 antibodies (137-1, 137-2,137-3) inhibiting CD73 enzyme activity on SK-OV-3 cells.
FIG. 13 shows a result diagram of CD73 internalization in SK-OV-3 cells mediated by humanized CD73, and MFI is the relative fluorescence intensity.
FIG. 14 shows that humanized 137 antibodies (137-1, 137-2, 137-3) inhibit tumor cells in the NSG-A375 model.
FIG. 15 shows the binding of humanized CD73 antibodies 137-4, 137-5, 137-6, 137-7, 137-8, 137-9 to recombinant human CD73 protein.
FIG. 16 shows the binding of humanized CD73 antibodies 137-4, 137-5, 137-6, 137-7, 137-8, 137-9 to recombinant monkey CD73 protein.
FIG. 17 shows an affinity diagram of CD73 antibodies (humanized CD73 antibody 137-4, 137-5, 137-6, 137-7, 137-8, 137-9, and MEDI-9447) and SK-OV-3 cells.
FIG. 18 shows an affinity diagram of humanized CD73 antibodies 137-4, 137-5, 137-6, 137-7, 137-8, 137-9 and CHOK1-cyno73 cells.
FIG. 19 shows the inhibition of humanized CD73 antibodies 137-4, 137-5, 137-6, 137-7, 137-8, 137-9 on rhCD73 enzyme activity.
FIG. 20 shows the inhibitory effect of humanized CD73 antibodies 137-4, 137-5, 137-6, 137-7, 137-8, 137-9 on CD73 enzyme activity on SK-OV-3 cells.
FIG. 21 shows CD73 internalization results of humanized CD73 antibodies 137-4, 137-5, 137-6, 137-7, 137-8, 137-9 mediated SK-OV-3 cells.

### Detailed description

Through extensive and deep research, after a large number of screening, the inventor accidentally obtains an anti-CD73 humanized antibody with excellent affinity. Specifically, according to the present invention, a high-specificity CD73 antibody is obtained by means of screening by using a phage display technology, and humanization is performed while both the similarity and the human body use frequency are taken into consideration. Through the binding experiment and affinity detection of the humanized antibody, the antibody binds specifically to the CD73 protein and CD73 positive cells of human and monkey, and has the effect of inhibiting the activity of CD73 protease. The antibodies of the present invention may also induce tumor cell internalization of CD73. In mouse experiments, the antibody of the present invention exhibits a better anti-tumor activity than the MEDI-9447 of AstraZeneca(AZ)/Medimmune. The present invention has been completed on the basis of this.

### Term

In order to make this disclosure easier to understand, certain technical and scientific terms are specifically defined below. Unless specifically defined otherwise herein, all other technical and scientific terms used herein have the meaning commonly understood by those skilled in the art to which the present invention belongs.

The amino acid three-letter code and one-letter code used in the present invention are as described in J.biol.chem,243,p3558(1968).

As used herein, the terms "administration" and "treatment" refer to exogenous drugs, therapeutic agents, diagnostic agents, or compositions for use in animals, humans, subjects, cells, tissues, organs, or biological fluids. "Administration" and "treatment" may refer to therapy, drug metabolism dynamics, diagnostics, studies, and experimental methods. The treatment of the cell includes the contact of the reagent with the cell, and the contact of the reagent with the fluid, the contact of the fluid with the cell. "Administration" and "treatment" also mean in vitro and ex vivo treatment by reagents, diagnostics, binding compositions, or by another cell. " Treatment ", when applied to a human, animal, or research subject, refers to therapeutic, prophylactic or prophylactic measures, studies and diagnostics; and it comprises contact of an anti-CD73 antibody with a human or animal, subject, cell, tissue, physiological compartment, or physiological fluid.

As used herein, the term "treatment" refers to the administration of an internal or external therapeutic agent comprising any one of the anti-CD73 antibodies and a composition thereof of the present invention to a patient having one or more disease symptoms for which the therapeutic agent is known to have a therapeutic effect. Generally, the amount of a therapeutic agent that effectively relieves one or more disease symptoms (therapeutically effective amount) is administrated to a patient.

As used herein, the terms "optional" or "optionally" mean that the subsequently described event or circumstance may occur but not necessarily. For example, "optionally contains 1-3 antibody heavy chain variable regions" refers to the specific sequence of the antibody heavy chain variable region can have, but not necessarily, may be 1, 2 or 3.

"Sequence identity" described in the present invention indicates the degree of identity between two nucleic acids or two amino acid sequences when optimally aligned and compared with appropriate mutations such as substitutions, additions or deletions. The sequence identity between the sequence described in the present invention and the sequence with identity to it can be at least 85%, 90%, or 95%, preferably at least 95%. Non-limiting examples include 85%, 86%, 87%, 88%, 89%, 90%, 91%, 92%, 93%, 94%, 95%, 96%, 97%, 98%, 99%, 100%.

### Antibody

As used herein, the term " antibody " refers to an immunoglobulin, which is a tetrapeptide chain structure formed by connecting two identical heavy chains and two identical light chains through an inter-chain disulfide bond. The amino acid composition and arrangement sequence of the immunoglobulin heavy chain constant region are different, so the antigenicity of the immunoglobulin heavy chain constant region is also different. Accordingly, immunoglobulins may be classified into five classes, or different types of immunoglobulins, ie, IgM, IgD, IgG, IgA and IgE. The heavy chain constant regions corresponding to different types of immunoglobulins are called α, δ, ε, γ and µ, respectively. IgG represents the most important class in immunoglobulin, which in turn can be divided into four subclasses: IgG1, IgG2, IgG3 and IgG4 due to differences in chemical and biological functions. The light chain is divided into κ or λ chains through different constant regions. The subunit structure and three-dimensional configuration of different classes of immunoglobulins are well known to those in the art.

The sequence of about 110 amino acids near the N terminal of the heavy chain and light chain of antibody changed greatly, which was the variable region (V region). The remaining amino acid sequence near the C terminal is relatively stable, which is the constant region (C region). The variable regions include three highly variable regions (HVRs) and four FR regions (FRs) with relatively conserved sequences. The 4 FR amino acid sequences are relatively conservative and do not directly participate in the binding reaction.

The three highly variable regions determine the specificity of the antibody, and are also referred to as complementarity determining regions (CDRs)). Each light chain variable region (LCVR) and the heavy chain variable region (HCVR) are composed of three CDR regions and four FR regions (frame regions), and sequentially arranged from the amino terminal to the carboxyl terminal are FR1, CDR1, FR2, CDR2, FR3, CDR3, and FR4. The three CDR regions of the light chain, ie the light chain highly variable region (LCDR), refer to LCDR1, LCDR2 and LCDR3; and the three CDR regions of the heavy chain, ie the heavy chain highly variable region (HCDR), refer to HCDR1, HCDR2 and HCDR3. The CDR amino acid residues of the LCVR and HCVR regions of the antibody or antigen-binding fragment of the invention conform in number and position to known Kabat numbering rules (LCDR1-3, HCDR2-3), or conform to the kabat and chothia numbering rules (HCDR1). Four FR regions of the variable regions of naturally occurring heavy and light chains are generally in a-sheet configuration, joined by the three CDRs forming a linking loop, and in some cases, may form a partical -sheet structure. The CDRs in each chain get close through the FR regions and together with the CDRs of the other chain form the antigen-binding site of the antibody. It can be determined which amino acids constitute the FR or CDR region by comparing the amino acid sequences of antibodies of the same type. Constant regions are not directly involved in the binding of antibodies to antigen, however, they exhibit different effector functions, such as participating in the antibody-dependent cytotoxicity of antibodies.

As used herein, the term "antigen-binding fragment" refers to a Fab fragment, a Fab' fragment, a F (ab')2 fragment, or a single Fv fragment with antigen-binding activity. Fv antibody contains antibody heavy chain variable region and light chain variable region, but no constant region, and has the smallest antibody fragment of all antigen binding sites. In general, Fv antibody further contains a polypeptide linker between the VH and VL domains and is capable of forming the structure required for antigen binding.

As used herein, the term "antigenic determinant" refers to a three-dimensional spatial site on an antigen that is not contiguous and is recognized by an antibody or antigen-binding fragment of the present invention.

The present invention includes not only intact antibodies, but also fragments of immunologically active antibodies or fusion proteins formed by antibodies with other sequences. Thus, the present invention also includes fragments, derivatives and analogs of the antibody.

In the present invention, the antibodies include murine, chimeric, humanized or full-human antibodies prepared with techniques well known to those skilled in the art. Recombinant antibodies, such as chimeric and humanized monoclonal antibodies, including human and non-human parts, can be prepared using DNA recombinant techniques well known in the art.

As used herein, the term "monoclonal antibody" refers to an antibody secreted from a single cell-derived clone. The monoclonal antibody is highly specific for a single antigenic epitope. The cells may be eukaryotic, prokaryotic or cloned cell strains of phage.

As used herein, the term "chimeric antibody" is an antibody molecule that is formed by splicing a V-region gene of a murine antibody and a C-region gene of a human antibody into a chimeric gene, then inserting a vector, and transfecting a host cell. The high specificity and affinity of the parent mouse antibody are retained, and the human Fc segment can effectively mediate the biological effect function.

As used herein, the term "humanized antibody" is a variable region modification form of a murine antibody of the present invention, having a CDR region derived (or substantially derived from) a non-human antibody (preferably a mouse monoclonal antibody), and an FR region and a constant region substantially derived from a human antibody sequence; ie. grafting the CDR region sequence of the mouse antibody onto different types of human-based antibody framework sequences. Because CDR sequences are responsible for most antibody-antigen interactions, recombinant antibodies that mimic specific naturally occurring antibody properties can be expressed by constructing an expression vector.

In the present invention, the antibody may be monospecific, bispecific, trispecific, or more multiple specificities.

In the present invention, the antibody of the present invention further includes a conservative variant thereof, which means a polypeptide formed by replacing at most 10, preferably at most 8, more preferably at most 5, and most preferably at most 3 amino acids with amnio acid of similar properties as compared with the amino acid sequence of the antibody of the present invention. These conservative variant polypeptides are best produced by amino acid substitution according to Table A.

**Table A.**

| Initial residue | Representative substitution | Preferred substitution |
|---|---|---|
| Ala (A) | Val; Leu; Ile | Val |
| Arg (R) | Lys; Gln; Asn | Lys |
| Asn (N) | Gln; His; Lys; Arg | Gln |
| Asp (D) | Glu | Glu |
| Cys (C) | Ser | Ser |
| Gln (Q) | Asn | Asn |
| Glu (E) | Asp | Asp |
| Gly (G) | Pro; Ala | Ala |
| His (H) | Asn; Gln; Lys; Arg | Arg |
| Ile (I) | Leu; Val; Met; Ala; Phe | Leu |
| Leu (L) | Ile; Val; Met; Ala; Phe | Ile |
| Lys (K) | Arg; Gln; Asn | Arg |
| Met (M) | Leu; Phe; Ile | Leu |
| Phe (F) | Leu; Val; Ile; Ala; Tyr | Leu |
| Pro (P) | Ala | Ala |
| Ser (S) | Thr | Thr |
| Thr (T) | Ser | Ser |
| Trp (W) | Tyr; Phe | Tyr |
| Tyr (Y) | Trp; Phe; Thr; Ser | Phe |
| Val (V) | Ile; Leu; Met; Phe; Ala | Leu |

### Anti-CD73 antibody

As use herein, the term "CD7" generally refers to natural or recombinant human CD73, and non-human homologues of human CD73.

The present invention provides an antibody with high specificity and high affinity against SEMA4D, which comprises a heavy chain and a light chain, wherein the heavy chain contains a heavy chain variable region (VH) amino acid sequence, and the light chain contains a light chain variable region (VL) amino acid sequence.

In the present invention, a high-quality human CD73 antigen immune mouse is selected, mouse immune cells are taken to construct a phage library, a special phage display technology is used, a single-chain antibody (scFv) is displayed on the surface of a phage, a CD73 antigen multi-round screening is performed to obtain an antibody sequence, and the antibody sequence is constructed by means of recombinant construction on a eukaryotic expression vector of an hIgG 1 framework, and mammalian cell expression obtains an anti-CD73 full-length antibody (ie, obtaining a human mouse chimeric antibody).

In a preferred embodiment of the present invention, the obtained human mouse chimeric anti-CD73 full-length antibody is a CQ137 antibody protein.. The obtained CQ137 antibody can bind to the CD73 molecule on the surface of tumors and inhibits the CD73 activity on human ovarian adenocarcinoma cells. The present invention has the potential to treat various CD73 overexpressing tumors.

Preferably, the CDRs of the heavy chain variable region (VH) are selected from the group consisting of:
VH-CDR1 as shown in SEQ ID NO.: 15,
VH-CDR2 as shown in SEQ ID NO.: 16, and
VH-CDR3 as shown in SEQ ID NO.: 17; and/or
the CDRs of the light chain variable region (VL) are selected from the group consisting of:
   VL-CDR1 as shown in SEQ ID NO: 18,
   VL-CDR2 as shown in SEQ ID NO: 19, and
   VL-CDR3 as shown in SEQ ID NO: 20.

Wherein, any one of the above amino acid sequences further includes a derivative sequence that is added, deleted, modified and/or substituted with at least one (such as 1-5, 1-3, preferably 1-2, more preferably 1) amino acid and has the affinity to bind to CD73.

In another preferred embodiment, the sequence with at least one amino acid added, deleted, modified and/or substituted in any of the above amino acid sequences is preferably an amino acid sequence having a homology of at least 80%, more preferably at least 85%, more preferably at least 90%, most preferably at least 95% to the above amino acid sequence.

The antibody of the present invention may be a double-chain or single-chain antibody, and may be selected from animal-derived antibodies, chimeric antibodies and humanized antibodies, more preferably be selected from humanized antibodies and human-animal chimeric antibodies, more preferably a fully humanized antibody.

The antibody derivatives of the present invention may be single chain antibodies, and/or antibody fragments, such as: Fab, Fab', (Fab')2 or other known antibody derivatives in the art, etc., as well as any one or several of IgA, IgD, IgE, IgG and IgM antibodies or other subtypes.

Wherein, the animal is preferably a mammal, such as a rat.

The antibody of the present invention may be a murine antibody, a chimeric antibody, a humanized antibody, a CDR grafted and/or modified antibody targeting human CD73.

In a preferred embodiment of the present invention, any one or several of the above SEQ ID NO.: 15, SEQ ID NO.:16 and SEQ ID NO.: 17, or a sequence with at least one amino acid added, deleted, modified and/or substituted thereof and with CD73 binding affinity is located in the CDR region of the heavy chain variable region (VH).

In a preferred embodiment of the present invention, any one or several of the above SEQ ID NO.: 18, SEQ ID NO.: 19 and SEQ ID NO.: 20, or a sequence with at least one amino acid added, deleted, modified and/or substituted thereof and with CD73 binding affinity is located in the CDR region of the light chain variable region (VL).

In a more preferred embodiment of the present invention, VH CDR1, CDR2 and CDR3 are independently selected from any one or several sequences of SEQ ID NO.: 15, SEQ ID NO.: 16 and SEQ ID NO.: 17 , and sequences with at least one amino acid added, deleted, modified and/or substituted thereof and with CD73 binding affinity; VL CDR1, CDR2 and CDR3 are independently selected from any one or several sequences of SEQ ID NO.: 18, SEQ ID NO.:19 and SEQ ID NO.: 20, and sequences with at least one amino acid added, deleted, modified and/or substituted thereof and with CD73 binding affinity.

In the above content of the present invention, the number of added, deleted, modified and/or substituted amino acids is preferably not more than 40% of the total number of amino acids in the original amino acid sequence, more preferably not more than 35%, more preferably 1-33% , more preferably 5-30%, more preferably 10-25%, more preferably 15-20%.

In the present invention, the number of added, deleted, modified and/or substituted amino acids is usually 1, 2, 3, 4, or 5, preferably 1-3, more preferably 1-2, optimally 1.

In another preferred embodiment, an anti-human CD73 (CQ137) antibody is provided
CQ137 light chain variable region (SEQ ID NO.:1): 
DILMTQSPSSMYASLGERVTITCKASQDINTYLTWFQQKPGKSPKTLIYR
ANILVDGVPSRFSGSGSGQDYSLTISSLEFEDMGIYYCLQYDEFPLTFGAGTK
LELKR
CQ137 heavy chain variable region (SEQ ID NO.: 2): 
EVQLQQSGPELVKPGASVKISCKASGYSFTGYYIHWVKQSHVKSLEWIG
RINPYNGATTYNQNFKDKASLTVDKSSSTAYMELHSLTSEDSAVYYCARFH
YGAPDYWGQGTTLTVSS
hIgG1 constant region amino acid sequence (SEQ ID NO.:3):
kappa chain constant region amino acid sequence (SEQ ID NO.:4):

TVAAPSVFIFPPSDEQLKSGTASVVCLLNNFYPREAKVQWKVDNALQSG

NSQESVTEQDSKDSTYSLSSTLTLSKADYEKHKVYACEVTHQGLSSPVTKSF
NRGEC

The present invention also provides the nucleotide sequence encoding the above amino acid:
CQ137 VH nucleotide sequence (SEQ ID NO.: 11): 
GAGGTTCAGCTGCAGCAGTCTGGACCTGAGCTGGTGAAGCCTGGGGC
TTCAGTGAAGATATCCTGCAAGGCTTCTGGTTACTCATTCACTGGCTACTA
CATCCACTGGGTGAAGCAAAGCCATGTAAAGAGCCTTGAGTGGATTGGA
CGTATTAATCCTTACAATGGTGCTACTACCTACAACCAGAATTTCAAGGA
CAAGGCCAGCTTGACTGTAGATAAGTCCTCCAGCACAGCCTACATGGAGC
TCCACAGCCTGACATCTGAGGACTCTGCAGTCTATTACTGTGCAAGATTC
CACTACGGTGCCCCTGACTACTGGGGCCAAGGCACCACTCTCACAGTCTC
CTCG
CQ137 VL nucleotide sequence (SEQ ID NO.: 12):

GATATTCTGATGACCCAATCTCCATCTTCCATGTATGCATCTCTAGGA
GAGAGAGTCACTATCACTTGCAAGGCGAGTCAGGACATTAATACCTATTT
AACTTGGTTCCAGCAGAAACCAGGGAAATCTCCTAAGACCCTGATCTATC
GTGCAAACATATTGGTAGATGGGGTCCCATCAAGGTTCAGTGGCAGTGGA
TCTGGGCAAGATTATTCTCTCACCATCAGCAGCCTGGAGTTTGAAGATAT
GGGAATTTATTATTGTCTACAGTATGATGAGTTTCCGCTCACGTTCGGTGC
TGGGACCAAGCTGGAGCTGAAACGT

The selected hIgG1 constant region nucleotide sequence is (SEQ ID NO: 13):

GCCTCCACCAAGGGCCCATCGGTCTTCCCCCTGGCACCCTCCTCCAA
GAGCACCTCTGGGGGCACAGCGGCCCTGGGCTGCCTGGTCAAGGACTACT
TCCCCGAACCGGTGACGGTGTCGTGGAACTCAGGCGCCCTGACCAGCGGC
GTGCACACCTTCCCGGCTGTCCTACAGTCCTCAGGACTCTACTCCCTCAGC
AGCGTGGTGACCGTGCCCTCCAGCAGCTTGGGCACCCAGACCTACATCTG
CAACGTGAATCACAAGCCCAGCAACACCAAGGTGGACAAGAAAGTTGAG
CCCAAATCTTGTGACAAAACTCACACATGCCCACCGTGCCCAGCACCTGA
ACTCCTGGGGGGACCGTCAGTCTTCCTCTTCCCCCCAAAACCCAAGGACA
CCCTCATGATCTCCCGGACCCCTGAGGTCACATGCGTGGTGGTGGACGTG
AGCCACGAAGACCCTGAGGTCAAGTTCAACTGGTACGTGGACGGCGTGG

AGGTGCATAATGCCAAGACAAAGCCGCGGGAGGAGCAGTACAACAGCAC
GTACCGGGTGGTCAGCGTCCTCACCGTCCTGCACCAGGACTGGCTGAATG
GCAAGGAGTACAAGTGCAAGGTCTCCAACAAAGCCCTCCCAGCCCCCATC
GAGAAAACCATCTCCAAAGCCAAAGGGCAGCCCCGAGAACCACAGGTGT
ACACCCTGCCCCCATCCCGGGATGAGCTGACCAAGAACCAGGTCAGCCTG
ACCTGCCTGGTCAAAGGCTTCTATCCCAGCGACATCGCCGTGGAGTGGGA
GAGCAATGGGCAGCCGGAGAACAACTACAAGACCACGCCTCCCGTGCTG
GACTCCGACGGCTCCTTCTTCCTCTACAGCAAGCTCACCGTGGACAAGAG
CAGGTGGCAGCAGGGGAACGTCTTCTCATGCTCCGTGATGCATGAGGCTC
TGCACAACCACTACACGCAGAAGAGCCTCTCCCTGTCTCCGGGTAAATGA

The selected kappa chain constant region nucleotide sequence is (SEQ ID NO: 14):

ACGGTGGCTGCACCATCTGTCTTCATCTTCCCGCCATCTGATGAGCAG
TTGAAATCTGGAACTGCCTCTGTTGTGTGCCTGCTGAATAACTTCTATCCC
AGAGAGGCCAAAGTACAGTGGAAGGTGGATAACGCCCTCCAATCGGGTA
ACTCCCAGGAGAGTGTCACAGAGCAGGACAGCAAGGACAGCACCTACAG
CCTCAGCAGCACCCTGACGCTGAGCAAAGCAGACTACGAGAAACACAAA
GTCTACGCCTGCGAAGTCACCCATCAGGGCCTGAGCTCGCCCGTCACAAA
GAGCTTCAACAGGGGAGAGTGTTAG

### Humanized anti-CD73 antibody

In 1986 Jones et al., the mouse monoclonal antibody heavy chain CDR is transplanted to the human antibody heavy chain skeleton region for the first time, and then assembled with the mouse monoclonal antibody light chain to form a complete antibody and maintain the affinity similar to that of the original mouse monoclonal antibody, thereby providing an idea for the development of the antibody humanized technology. In 1989 Queen et al., the anti-CD25 humanized antibody was successfully constructed by means of CDR transplantation. The method uses a human antibody Eu skeleton region for humanization, and retains the murine antibody amino acid at some sites of the skeleton region to maintain affinity. In 1992, Presta et al. reported a method for successfully constructing a humanized method by taking a consensus sequence as a template for CDR transplantation. In 1994, Pedersen et al. reported that the antibody is humanized with a method of resurfacing. In 1994 Hsiao et al., a humanized method for CDR transplantation in a human antibody Germline sequence skeleton region is reported. In 1994, Jespers et al. successfully constructed the humanization method using the method of phage library.

The present invention also provides a humanized antibody with high specificity and high affinity for CD73.

In another preferred example, said sequence further comprises a sequence formed by addition, deletion, modification and/or substitution of at least one amino acid sequence, preferably an amino acid sequence having a homology of at least 80%, preferably at least 85%, more preferably at least 90%, optimally at least 95%.

The antibody of the present invention may be a double-chain or single-chain antibody, and may be preferably a fully humanized antibody.

The antibody derivatives of the present invention may be single chain antibodies, and/or antibody fragments, such as: Fab, Fab', (Fab')₂ or other known antibody derivatives in the art, etc., as well as any one or several of IgA, IgD, IgE, IgG and IgM antibodies or other subtypes.

The antibody of the present invention may be a humanized antibody, a CDR grafted and/or modified antibody targeting human CD73.

In the above content of the present invention, the number of added, deleted, modified and/or substituted amino acids is preferably not more than 40% of the total number of amino acids in the original amino acid sequence, more preferably not more than 35%, more preferably 1-33% , more preferably 5-30%, more preferably 10-25%, more preferably 15-20%.

### Preparation of Antibodies

Any method suitable for producing monoclonal antibodies can be used to produce the CD73 antibody of the present invention. For example, an animal may be immunized with a linked or naturally occurring CD73 protein or a fragment thereof. Suitable immune inoculation methods may be used, including adjuvants, immunostimulatory agents, repeated enhanced immune inoculation, and one or more approaches may be used.

Any suitable form of CD73 can be used as an immunogen (antigen) for producing a non-human antibody specific to CD73 and screening the biological activity of the antibody. The immunogen may be used alone or in combination with one or more immunogenic enhancers known in the art. The immunogen may be purified from a natural source, or produced in genetically modified cells. The DNA encoding the immunogen may be a genomic or non-genome (eg, cDNA) on the source. DNA encoding an immunogen may be expressed using a suitable genetic vector including, but not limited to, an adenovirus vector, a baculovirus vector, a plasmid, and a non-viral vector.

Humanized antibodies may be selected from any kind of immuno globulins, including IgM, IgD, IgG, IgA, and IgE. Likewise, any class of light chains may be used in the compounds and methods herein. Specifically, κ, λ chains, or variants thereof are useful in the compounds and methods of the invention.

An exemplary method of preparing the CD73 antibody of the present invention is described in Example 1.

The sequence of the DNA molecule for the antibody or a fragment thereof according to the present invention can be obtained by conventional techniques, for example, methods such as PCR amplification or genomic library screening. In addition, the sequences encoding light chain and heavy chain can be fused together, to form a single-chain antibody.

Once a relevant sequence is obtained, recombination methods can be used to obtain the relevant sequence in large quantities. This is usually carried out by cloning the sequence into a vector, transforming a cell with the vector, and then separating the relevant sequence from the proliferated host cell by conventional methods.

In addition, a relevant sequence can be synthesized artificially, especially when the fragment is short in length. Usually, several small fragments are synthesized first, and then are linked together to obtain a fragment with a long sequence. The DNA sequence can then be introduced into various existing DNA molecules (or, for example, vectors) and cells known in the art.

The term "nucleic acid molecule" refers to a DNA molecule and an RNA molecule. The nucleic acid molecules may be single-stranded or double-stranded, but are preferably double-stranded DNA. When a nucleic acid is placed in a functional relationship with another nucleic acid sequence, the nucleic acid is "effectively connected". For example, if the promoter or enhancer affects the transcription of the coding sequence, the promoter or enhancer is effectively connected to the coding sequence.

The term "vector" refers to a nucleic acid molecule capable of transporting another nucleic acid connected thereto. In one embodiment, the vector is a "plasmid" which refers to a cyclic double-stranded DNA loop that can connect an additional DNA segment to it.

The present invention also relates to a vector comprising the appropriate DNA sequence as described above and an appropriate promoter or control sequence. These vectors can be used to transform suitable host cells to enable them to express protein.

The term "host cell" refers to a cell into which an expression vector has been introduced. Host cells may be prokaryotic cells, such as bacterial cells; or lower eukaryotic cells, such as yeast cells; or higher eukaryotic cells, such as plant or animal cells (such as mammalian cells).

The steps of transforming host cells with recombinant DNA according to the present invention may be performed by techniques well known in the art. The obtained transformant can be cultured by conventional methods, and the transformants express the polypeptide encoded by the gene of the present invention. According to the host cells used, culture is performed under suitable conditions with conventional culture media.

In general, under conditions suitable for expression of the antibody according to the present invention, the host cell obtained is cultured. Then, the antibody according to the present invention is purified by using conventional immunoglobulin purification steps, for example, the conventional separation and purification means well known to those skilled in the art, such as protein A-Sepharose, hydroxyapatite chromatography, gel electrophoresis, dialysis, ion exchange chromatography, hydrophobic chromatography, molecular sieve chromatography or affinity chromatography.

The monoclonal antibody obtained can be identified by conventional means. For example, the binding specificity of a monoclonal antibody can be determined by immunoprecipitation or an in vitro binding assay (such as radioimmunoassay (RIA) or enzyme-linked immunosorbent assay (ELISA)).

### Antibody-Drug Conjugate (ADC)

The present invention also provides an antibody-drug conjugate (ADC) based on the antibody according to the present invention.

Typically, the antibody-drug conjugate comprises the antibody and an effector molecule, wherein the antibody is conjugated to the effector molecule, and chemical conjugation is preferred. Preferably, the effector molecule is a therapeutically active drug. In addition, the effector molecule may be one or more of a toxic protein, a chemotherapeutic drug, a small-molecule drug or a radionuclide.

The antibody according to present invention and the effector molecule may be coupled by a coupling agent. Examples of the coupling agent may be any one or more of a non-selective coupling agent, a coupling agent utilizing a carboxyl group, a peptide chain, and a coupling agent utilizing a disulfide bond. The non-selective coupling agent refers to a compound that results in a linkage between an effector molecule and an antibody via a covalent bond, such as glutaraldehyde, etc. The coupling agent utilizing a carboxyl group may be any one or more of cis-aconitic anhydride coupling agents (such as cis-aconitic anhydride) and acyl hydrazone coupling agents (the coupling site is acyl hydrazone).

Certain residues on an antibody (such as Cys or Lys, etc.) are used to link a variety of functional groups, including imaging agents (such as chromophores and fluorophores), diagnostic agents (such as MRI contrast agents and radioisotopes), stabilizers (such as poly(ethylene glycol)) and therapeutic agents. An antibody can be conjugated to a functional agent to form a conjugate of the antibody-functional agent. A functional agent (e.g. a drug, a detection reagent, a stabilizer) is conjugated (covalently linked) to an antibody. A functional agent can be linked to an antibody either directly or indirectly via a linker.

Antibodies can be conjugated to drugs to form antibody-drug conjugates (ADCs). Typically, an ADC comprises a linker between a drug and an antibody. The linker can be a degradable or non-degradable linker. Typically, degradable linkers are easily degraded in an intracellular environment, for example, the linker is degraded at the target site, thereby releasing the drug from the antibody. Suitable degradable linkers include, for example, enzyme-degradable linkers, including peptidyl-containing linkers that can be degraded by protease (e.g. lysosomal protease or endosomal protease) in a cell, or sugar linkers, for example, glucuronide-containing linkers that can be degraded by glucuronidase. Peptidyl linkers may include, for example, dipeptides, such as valine-citrulline, phenylalanine-lysine or valine-alanine. Other suitable degradable linkers include, for example, pH sensitive linkers (e.g. linkers that are hydrolyzed at a pH of below 5.5, such as hydrazone linkers) and linkers that are degraded under reducing conditions (e.g. disulfide-bond linkers). A non-degradable linker typically releases a drug under conditions that the antibody is hydrolyzed by protease.

Prior to linkage to an antibody, a linker has a reactive group capable of reacting with certain amino acid residues, and the linkage is achieved by the reactive group. A thiol-specific reactive group is preferred, and includes, for example, a maleimide compound, a halogenated (e.g. iodo-, bromo- or chloro-substituted) amide; a halogenated (e.g. iodo-, bromo- or chloro-substituted) ester; a halogenated (e.g. iodo-, bromo- or chloro-substituted) methyl ketone, a benzyl halide (e.g. iodide, bromide or chloride); vinyl sulfone, pyridyl disulfide; a mercury derivative such as 3,6-di-(mercurymethyl)dioxane, wherein the counter ion is CH3COO-, Cl- or NO3-; and polymethylene dimethyl sulfide thiosulfonate. The linker may include, for example, a maleimide linked to an antibody via thiosuccimide.

A drug may be any cytotoxic, cytostatic or immunosuppressive drug. In an embodiment, an antibody is linked to a drug via a linker, and the drug has a functional group that can form a bond with the linker. For example, a drug may have an amino group, a carboxyl group, a thiol group, a hydroxyl group, or a ketone group that can form a bond with a linker. When a drug is directly linked to a linker, the drug has a reactive group before being linked to an antibody.

Useful drugs include, for example, anti-tubulin drugs, DNA minor groove binding agents, DNA replication inhibitors, alkylating agents, antibiotics, folic acid antagonists, antimetabolites, chemotherapy sensitizers, topoisomerase inhibitors, vinca alkaloids, etc. In the present invention, a drug-linker can be used to form an ADC in a simple step. In other embodiments, a bifunctional linker compound can be used to form an ADC in a two-step or multi-step process. For example, a cysteine residue is reacted with the reactive moiety of a linker in a first step, and then the functional group on the linker is reacted with a drug in the subsequent step, so as to form an ADC.

In general, the functional group on a linker is selected so that it can specifically react with the suitable reactive group on a drug moiety. As a non-limiting example, an azide-based moiety can be used to specifically react with the reactive alkynyl group on a drug moiety. The drug is covalently bound to the linker by 1,3-dipolar cycloaddition between the azide and alkynyl group. Other useful functional groups include, for example, ketones and aldehydes (suitable for reacting with hydrazides and alkoxyamines), phosphines (suitable for reacting with azides); isocyanates and isothiocyanates (suitable for reacting with amines and alcohols); and activated esters, for example, N-hydroxysuccinimide esters (suitable for reacting with amines and alcohols). These and other linkage strategies, for example, those described in Bioconjugation Technology (2nd Edition (Elsevier)), are well known to those skilled in the art. Those skilled in the art could understand that when a complementary pair of reactive functional groups are selected for a selective reaction between a drug moiety and a linker, each member of the complementary pair can be used for the linker, and can also be used for the drug.

### Antibody preparation

The antibodies have different stability in different preparation buffers, show changes in charge heterogeneity, degradation, polymerization of antibody molecules, etc., and the changes of these quality properties are related to the physical and chemical properties of the antibody. Therefore, in the antibody drug development process, the preparation buffer suitable for itself needs to be screened according to the physical and chemical properties of different antibodies. At present, the commonly used antibody preparation buffer system has a phosphate buffer solution, a citric acid buffer solution, a histidine buffer solution, etc., and salt ions or sorbitol, trehalose, sucrose and other excipients with different concentrations are added according to the properties of the antibody, and a proper amount of surfactants such as Tween 20 or Tween 80 are added to maintain the stability of the antibody.

The antibody-drug combination preparation of the present invention can effectively inhibit side reactions such as aggregation precipitation, hydrolysis, oxidation and deamidation of the humanized antibody of the present inventors, and can effectively improve the stability of the product under the conditions of pressurization (high temperature, strong light irradiation, freeze thawing, etc.), acceleration, and long-term refrigeration.

### Pharmaceutical composition

The present invention also provides a composition. In the preferred examples, the composition is a pharmaceutical composition comprising the antibody, or an active fragment, a fusion protein or an ADC thereof, or a corresponding CAR-T cell, and a pharmaceutically acceptable carrier. Typically, these substances may be formulated in a non-toxic, inert and pharmaceutically acceptable aqueous carrier medium, wherein the pH is typically about 5-8 and preferably about 6-8, although the pH may vary depending on the nature of the substance being formulated and the condition to be treated. The formulated pharmaceutical composition may be administered by conventional routes, including (but not limited to) intratumoral, intraperitoneal, intravenous, or topical administration.

The antibody of the present invention can also be used for cell therapy by expressing the nucleotide sequence in the cell. For example, the antibody is used for chimeric antigen receptor T cell immunotherapy (CAR-T) and the like.

The pharmaceutical composition according to the present invention can be directly used for binding to a CD73 protein molecule, and thus can be used for preventing and treating CD73-related diseases. In addition, other therapeutic agents may be used at the same time.

The pharmaceutical composition of the present invention contains a safe and effective amount (e.g., 0.001-99wt%, preferably 0.01-90wt%, more preferably 0.1-80wt%) of the above-mentioned single domain antibody of the present invention (or the conjugate thereof) and a pharmaceutically acceptable carrier or excipient. Such carriers include, but are not limited to, saline, buffer, glucose, water, glycerol, ethanol, and a combination thereof. The pharmaceutical preparation should be matched to the method of administration. The pharmaceutical composition of the present invention can be prepared in the form of injection, for example, prepared by a conventional method using physiological saline or an aqueous solution containing glucose and other adjuvants. The pharmaceutical composition such as an injection and solution should be manufactured under sterile conditions. The dosage of the active ingredient is a therapeutically effective amount, for example, about 1 µg/kg body weight per day to about 5 mg/kg body weight. In addition, the polypeptide according to the present invention may also be used in combination with an additional therapeutic agent.

When a pharmaceutical composition is used, a safe and effective amount of the pharmaceutical composition is administered to a mammal, wherein the safe and effective amount is typically at least about 10 µg/kg body weight, and in most cases no more than about 50 mg/kg body weight, preferably about 10 µg/kg body weight to about 20 mg/kg body weight. Of course, the particular dose should also depend on various factors, such as the route of administration, patient healthy status, which are well within the skills of an experienced physician.

### Use for detection and the kits

The antibodies of the present invention can be used in detection applications, for example, for detecting a sample to provide diagnostic information.

In the present invention, the sample includes a cell sample, a tissue sample, and a biopsy sample. The term 'biopsy' used in the present invention should include all types of biopsies known to those skilled in the art. Therefore, the biopsy used in the present invention can include tissue samples prepared, for example, through endoscopic methods or organ puncture or needle biopsy.

Samples for use in the present invention include fixed or preserved cell or tissue samples.

The present invention also provides a kit containing the antibody (or fragment thereof) of the present invention. In a preferred embodiment of the present invention, the kit further comprises a container, instructions for use, and a buffer, etc. In a preferred embodiment, the antibody of the present invention may be immobilized on a detection plate.

### The main advantages of the present invention include:

(1) The humanized antibody of the present invention has better security compared with a murine and chimeric antibody;
(2) The antibody of the present invention has higher affinity by competitive screening;
(3) The antibody of the present invention is derived from phage display technology, and has stronger diversity than hybridoma technology.

The invention is further illustrated below in conjunction with specific embodiments. The present disclosure is further set forth below with reference to specific embodiments. The conditions of the experimental methods not specifically indicated in the following examples are usually in accordance with conventional conditions as described in Sambrook et al., Molecular Cloning: A Laboratory Manual (New York: Cold Spring Harbor Laboratory Press, 1989), or according to the conditions recommended by the manufacturer. Percentages and parts are by weight unless otherwise stated.

### Example 1 Preparation of anti-human CD73 monoclonal antibody

The present example mainly describes mouse immunization and phage display to obtain an anti-human CD73 single-chain antibody sequence. The recombinant human CD73 protein (C446, Novoprotein) was used to immunize Balb/C mice, and after the first immunization, booster immunization was performed every 14 days for a total of 4 times, and mouse serum was collected for antibody titer evaluation. An effective mouse was evaluated to take B cells, RNA was extracted for reverse transcription, and a phage display library was constructed. The recombinant human CD73 protein was packaged in plates according to 1-5 µg/ml, the displayed phage was added, phage library screening was performed, the screened unwashed phage was collected and infected with host bacteria, a first round of screening library was obtained, and second rounds and third rounds of screening were performed according to the method. After the screening was completed, phage-ELISA identification was performed, and the positive one was sequenced to abtain anti-CD73 candidate sequences. CQ137 VH and VL were respectively constructed on a eukaryotic expression vector containing an hIgG1-kappa constant region, the freestyle 293F cell was transfected, supernatant was collected after 3-7 days of culture, and Protein A column was used for purification to obtain a CQ137 antibody protein.
CQ137 light chain variable region (SEQ ID NO.:1) 
DILMTQSPSSMYASLGERVTITCKASQDINTYLTWFQQKPGKSPKTLIYR
ANILVDGVPSRFSGSGSGQDYSLTISSLEFEDMGIYYCLQYDEFPLTFGAGTK
LELKR
CQ137 heavy chain variable region (SEQ ID NO.:2) 
EVQLQQSGPELVKPGASVKISCKASGYSFTGYYIHWVKQSHVKSLEWIG
RINPYNGATTYNQNFKDKASLTVDKSSSTAYMELHSLTSEDSAVYYCARFH
YGAPDYWGQGTTLTVSS
hIgG1 constant region amino acid sequence (SEQ ID NO.: 3)
kappa chain constant region amino acid sequence (SEQ ID NO.: 4)

TVAAPSVFIFPPSDEQLKSGTASVVCLLNNFYPREAKVQWKVDNALQSG
NSQESVTEQDSKDSTYSLSSTLTLSKADYEKHKVYACEVTHQGLSSPVTKSF
NRGEC

### Example 2 Affinity detection of CQ137 antibody

This example mainly describes affinity detection of CQ137 with human and monkey recombinant CD73 proteins, as well as affinity detection of human CD73 positive cells (SK-OV-3, human ovarian adenocarcinoma cells), and monkey CD73 cell strains (CHOK1-cynod 73).

### (1) Affinity to human CD73 protein

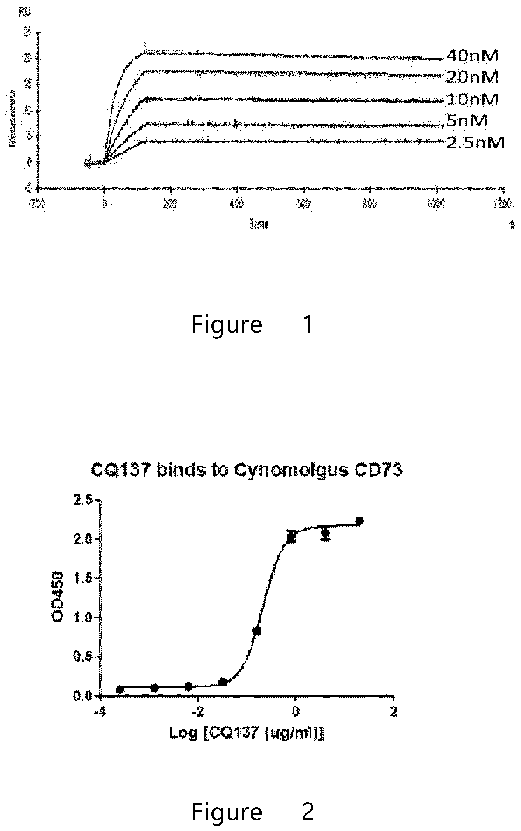

The affinity of CQ137 to recombinant hCD73 protein was determined by Biacore, CQ137 was cured. CD73 protein was diluted by double gradient (2.5nM-40nM), and the obtained affinity map was shown in FIG.1.

The affinity data is shown in table 1 below:

**Table 1. CQ137 affinity**

| KD(M) | ka(1/Ms) | kd(1/s) |
|---|---|---|
| 8.653E-11 | 6.977E+5 | 6.037E-5 |

The results show that the affinity of CQ137 to rhCD73 is 8.653 × 10⁻¹¹M.

### (2) Affinity to monkey CD73 Protein

The recombinant monkey CD73 protein was packaged in plates according to a 3 µg/mL and a gradient diluted CQ137 was added. The combination of CQ137 and the recombinant monkey CD73 was detected by an ELISA method, and the result is shown in FIG. 2.

The results showed that the calculated EC50 value for CQ137 binding to recombinant monkey CD73 was 0.217 µg/ml.

### (3) Binding to human CD73 positive cells

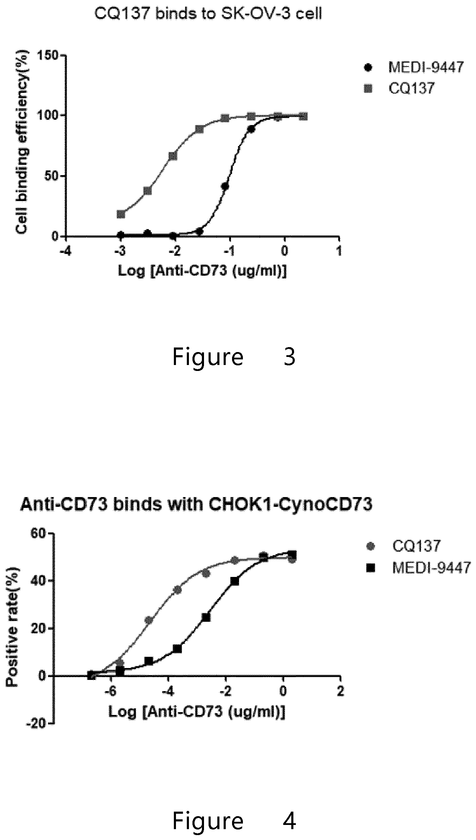

4×10⁵ cells SK-OV-3 were taken, triple gradient (0.009-20 µg/ml) dilution of CQ137 protein was added, incubated for 1 h, washed three times with PBS, Anti-hFC-APC (purchased from Jackson immunology) was added, and detected by flow cytometry. S-curves were plotted and the results are shown in Figure 3.

The results show that EC50 of CQ137 combined with SK-OV-3 cells is 0.5883ng/mL, and EC50 of MEDI-9447 combined with SK-OV-3 cells is 9.627ng/mL. Therefore, the combination of CQ137 and SK-OV-3 cells is superior to MEDI-9447.

### (4) Binding to monkey CD73 positive cells

The full length of monkey CD73 was constructed on a eukaryotic expression vector containing NEO resistance, CHO-K1 cells were transfected, G418 was used for screening to obtain a CHOK1-cynoCD73 cell strain. 4×10⁵ CHOK1-cynoCD73 cells were taken and a gradient diluted CQ137 protein was added, after incubation was performed for 1 h, the cells were washed with PBS for three times, Anti-Hail-APC (purchased from Jackson Immunology) was added, and detected by flow cytometry. S-curves were plotted and the results are shown in Figure 4.

The results show that EC50 of CQ137=0.09991ng/mL, and EC50 of MEDI-9447=4.119 ng/mL. Therefore, the binding of CQ137 to CHOK1-cynoCD73 cells is superior to MEDI-9447.

### Example 3 CQ 137-Mediated CD73 Enzyme Activity Inhibition Detection

In this embodiment, CQ137 is mainly shown to inhibit CD73 protein and cell enzyme activity. Specifically,

### (1) CD73 protease activity inhibition

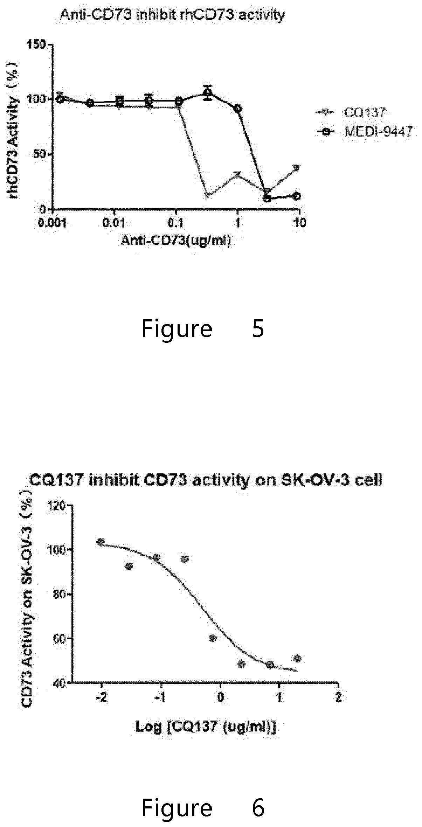

The CD73 protein was diluted to a working concentration of 5 µg/mL, a CD73 antibody diluted with a triple gradient (0.001 -10 µg/mL) was added, incubation was performed at 37°C for 15 min, a mixed solution of 1nM ATP and AMP was added, incubation was performed at 37°C for 30 min, a CellTiter-GLo detection reagent (purchased from promega) was added in equal volumes, a self-luminescence value was read by an ELISA instrument, and the enzyme activity was calculated as 100% without the addition of antibody, and the change in rhCD73 activity was obtained, see FIG.5.

The results show that CQ137 is superior to the control molecule MEDI-9447 in inhibiting the CD73 protease activity.

### (2) Inhibition of CD73 enzyme activity on human SK-OV-3 cells

5 × 10⁴ SK-OV-3 cells were taken, triple gradient (0.009-20 µg/mL) dilution of antibody was added, incubated at 37°C for 15 min, 1nM AMP was added at 37°C for 2h, 1nM ATP was added, and a CellTiter-GLo detection reagent was added immediately, a self-luminescence value was read by an ELISA instrument, and the enzyme activity was calculated as 100% without the addition of antibody, and the change in rhCD73 activity was obtained, see FIG.6.

The results show that CQ137 can inhibit the CD73 enzyme activity on SK-OV-3 cells, and IC50 of CQ137 is 0.3708µg/mL.

### (3) Inhibition of CD73 enzyme activity on CHOK1-cynoCD73 Cells

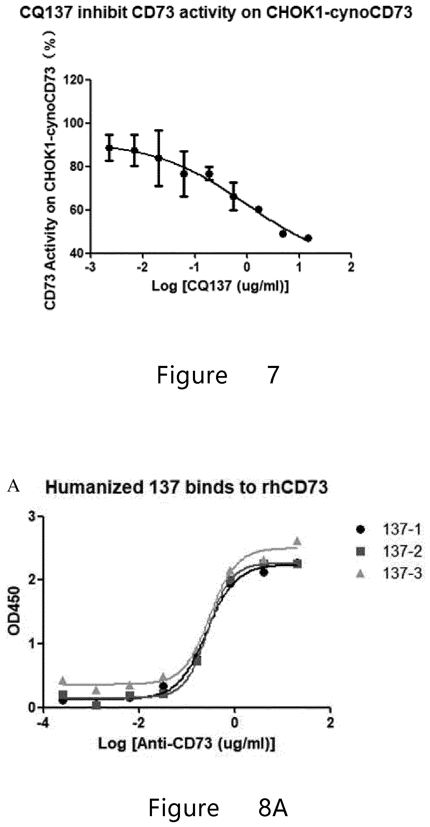

5 × 10⁴ CHOK1-cynoCD73 cells were taken, triple gradient (0.003-20 µg/mL) dilution of antibody was added, incubated at 37°C for 15 min, 1nM AMP was added at 37°C for 2h, 1nM ATP was added, and a CellTiter-GLo detection reagent was added immediately, a self-luminescence value was read by an ELISA instrument, and the enzyme activity was calculated as 100% without the addition of antibody, and the change in rhCD73 activity was obtained and shown in FIG.7.

The results show that CQ137 can inhibit the CD73 enzyme activity on the CHOK1-cynoCD73 cells, and the IC50 of CQ137 is 0.9899µg/ml.

### Example 4 CQ137 Humanization

By utilizing structural simulation and rational design, a human frame closest to CQ137 was analyzed to obtain a series of humanized antibodies. The VH and VL of the humanized sequence were respectively constructed on a vector containing hIgG1 and kappa light chain constant regions, the Freestyle 293F cells were transfected, supernatant was collected, and the required antibody protein was obtained after Protein A column purification.

The sequences of the 9 humanized CQ137 monoclonal antibodies (137-1, 137-2, 137-3, 137-4, 137-5, 137-6, 137-7, 137-8, 137-9) obtained are shown below:
The humanized CQ137 monoclonal antibody (137-1) heavy chain VH amino acid sequence is (SEQ ID NO.: 5) 
QVQLVQSGAEVKKPGASVKVSCKASGYTFTGYYIHWVRQAPGQGLEW
MGRINPYNGATTYNQNFKDRVTMTVDKSTSTAYMELRSLRSDDTAVYYCA
RFHYGAPDYWGQGTLVTVSS
The humanized CQ137 monoclonal antibody (137-1) light chain VL amino acid sequence is (SEQ ID NO.: 6) 
DIQMTQSPSSLSASVGDRVTITCKASQDINTYLTWFQQKPGKAPKTLIYR
ANILVDGVPSRFSGSGSGQDYTLTISSLQPEDFATYYCLQYDEFPLTFGGGTK
VEIKR
The humanized CQ137 monoclonal antibody (137-2) heavy chain VH amino acid sequence is (SEQ ID NO.: 7) 
QVQLVQSGAEVKKPGASVKVSCKASGYSFTGYYIHWVRQAPGQGLEW
MGRINPYNGATTYNQNFKDRVTMTVDKSTSTAYMELRSLRSDDTAVYYCA
RFHYGAPDYWGQGTLVTVSS
The humanized CQ137 monoclonal antibody (137-2) light chain VL amino acid sequence is (SEQ ID NO.: 8) 
DIQMTQSPSSLSASVGDRVTITCKASQDINTYLTWFQQKPGKAPKSLIYR
ANILVDGVPSRFSGSGSGTDFTLTISSLQPEDFATYYCLQYDEFPLTFGGGTK
VEIKR
The humanized CQ137 monoclonal antibody (137-3) heavy chain VH amino acid sequence is (SEQ ID NO.: 9) 
QVQLVQSGAEVKKPGSSVKVSCKASGYSFTGYYIHWVRQAPGQGLEW
MGRINPYNGATTYNQNFKDRVTITVDKSTSTAYMELSSLRSEDTAVYYCARF
HYGAPDYWGQGTLVTVSS
The humanized CQ137 monoclonal antibody (137-3) light chain VL amino acid sequence is (SEQ ID NO.: 10) 
DIQMTQSPSSLSASVGDRVTITCKASQDINTYLTWYQQKPGKAPKTLIYR
ANILVDGVPSRFSGSGSGTDFTLTISSLQPEDFATYYCLQYDEFPLTFGGGTK
VEIKR
The humanized CQ137 monoclonal antibody (137-4) heavy chain VH amino acid sequence is (SEQ ID NO.: 34) 
QVQLVQSGAEVKKPGSSVKVSCKASGYSFTGYYIHWVRQAPGQGLEW
MGRINPYNGATTYNQNFKDRVTITVDKSTSTAYMELSSLRSEDTAVYYCARF
HYGAVDYWGQGTLVTVSS
The humanized CQ137 monoclonal antibody (137-4) light chain VL amino acid sequence is (SEQ ID NO.: 40) 
DIQMTQSPSSLSASVGDRVTITCKASQDINTYLTWYQQKPGKAPKTLIYR
ANILVDGVPSRFSGSGSGTDFTLTISSLQPEDFATYYCLQYEEFPLTFGGGTKV
EIKR
The humanized CQ137 monoclonal antibody (137-5) heavy chain VH amino acid sequence is (SEQ ID NO.: 35) 
QVQLVQSGAEVKKPGSSVKVSCKASGYSFTGYYIHWVRQAPGQGLEW
MGRINPYNGATTYNQNFKDRVTITVDKSTSTAYMELSSLRSEDTAVYYCARF
HYGAADYWGQGTLVTVSS
The humanized CQ137 monoclonal antibody (137-5) light chain VL amino acid sequence is (SEQ ID NO.: 41) 
DIQMTQSPSSLSASVGDRVTITCKASQDINTYLTWYQQKPGKAPKTLIYR
ANILVDGVPSRFSGSGSGTDFTLTISSLQPEDFATYYCLQYNEFPLTFGGGTK
VEIKR
The humanized CQ137 monoclonal antibody (137-6) heavy chain VH amino acid sequence is (SEQ ID NO.: 36) 
QVQLVQSGAEVKKPGSSVKVSCKASGYSFTGYYIHWVRQAPGQGLEW
MGRINPYNGATTYNQNFKDRVTITVDKSTSTAYMELSSLRSEDTAVYYCARF
HYGAPEYWGQGTLVTVSS
The humanized CQ137 monoclonal antibody (137-6) light chain VL amino acid sequence is (SEQ ID NO.: 42) 
DIQMTQSPSSLSASVGDRVTITCKASQDINTYLTWYQQKPGKAPKTLIYR
ANILVDGVPSRFSGSGSGTDFTLTISSLQPEDFATYYCLQYAEFPLTFGGGTK
VEIKR
The humanized CQ137 monoclonal antibody (137-7) heavy chain VH amino acid sequence is (SEQ ID NO.: 37) 
QVQLVQSGAEVKKPGSSVKVSCKASGYSFTGYYIHWVRQAPGQGLEW
MGRINPYNGATTYNQNFKDRVTITVDKSTSTAYMELSSLRSEDTAVYYCARF
HYGAPNYWGQGTLVTVSS
The humanized CQ137 monoclonal antibody (137-7) light chain VL amino acid sequence is (SEQ ID NO.: 43) 
DIQMTQSPSSLSASVGDRVTITCKASQDINTYLTWYQQKPGKAPKTLIYR
ANILVDGVPSRFSGSGSGTDFTLTISSLQPEDFATYYCLQYDDFPLTFGGGTK
VEIKR
The humanized CQ137 monoclonal antibody (137-8) heavy chain VH amino acid sequence is (SEQ ID NO.: 38) 
QVQLVQSGAEVKKPGSSVKVSCKASGYSFTGYYIHWVRQAPGQGLEW
MGRINPYNGATTYNQNFKDRVTITVDKSTSTAYMELSSLRSEDTAVYYCARF
HYGAPDFWGQGTLVTVSS
The humanized CQ137 monoclonal antibody (137-8) light chain VL amino acid sequence is (SEQ ID NO.: 44) 
DIQMTQSPSSLSASVGDRVTITCKASQDINTYLTWYQQKPGKAPKTLIYR
ANILVDGVPSRFSGSGSGTDFTLTISSLQPEDFATYYCLQYDQFPLTFGGGTK
 VEIKR
The humanized CQ137 monoclonal antibody (137-9) heavy chain VH amino acid sequence is (SEQ ID NO.: 39) 
QVQLVQSGAEVKKPGSSVKVSCKASGYSFTGYYIHWVRQAPGQGLEW
MGRINPYNGATTYNQNFKDRVTITVDKSTSTAYMELSSLRSEDTAVYYCARF
HYGAPDLWGQGTLVTVSS
The humanized CQ137 monoclonal antibody (137-9) light chain VL amino acid sequence is (SEQ ID NO.: 45)

DIQMTQSPSSLSASVGDRVTITCKASQDINTYLTWYQQKPGKAPKTLIYR
ANILVDGVPSRFSGSGSGTDFTLTISSLQPEDFATYYCLQYDAFPLTFGGGTK
VEIKR

The underlined region is CDRs (IMGT definition), 137-1, 137-2, 137-3, 137-4, 137-5, 137-6, 137-7, 137-8, 137-9 are protein numbers of nine humanized CQ137 antibodies, respectively. The third amino acid (Thr) in the heavy chain VH-CDR1: GYTFTGYY (SEQ ID NO.:21) of the humanized antibody 137-1 is different from the third amino acid (Ser) in the heavy chain VH-CDR1: GYSFTGYY (SEQ ID NO.:15) of the CQ137 antibody. CDR regions of humanized antibodies 137-2, 137-3 are the same as CQ137 antibodies. The FR region of the humanized antibody is replaced on the basis of the CQ137 antibody. The CDR of the partially humanized antibody is replaced on the basis of the CQ137 antibody.

### Example 5 CQ137 Humanized Affinity Evaluation

The present example mainly shows affinity of humanized CD73 antibody to recombinant human and monkey CD73 protein, and affinity of CD73 positive cell SK-OV-3 cells and CHOK1-cynoCD73 cells, respectively. Specifically,

### (1) binding to recombinant human CD73 protein

The recombinant human CD73 protein was packaged in plates according to a 3 µg/mL and a gradient diluted humanized CD73 antibodies (137-1, 137-2, 137-3) were added. The combination of antibody and the recombinant human CD73 was detected by an ELISA method, and the result is shown in FIG. 8A.

The results are shown in Table 2.

**Table 2. EC50 for binding of humanized CD73 antibody to recombinant human CD73 protein**

| Humanized CD73 antibody No. | 137-1 | 137-2 | 137-3 |
|---|---|---|---|
| EC50 (µg/ml) | 0.2581 | 0.2746 | 0.3307 |

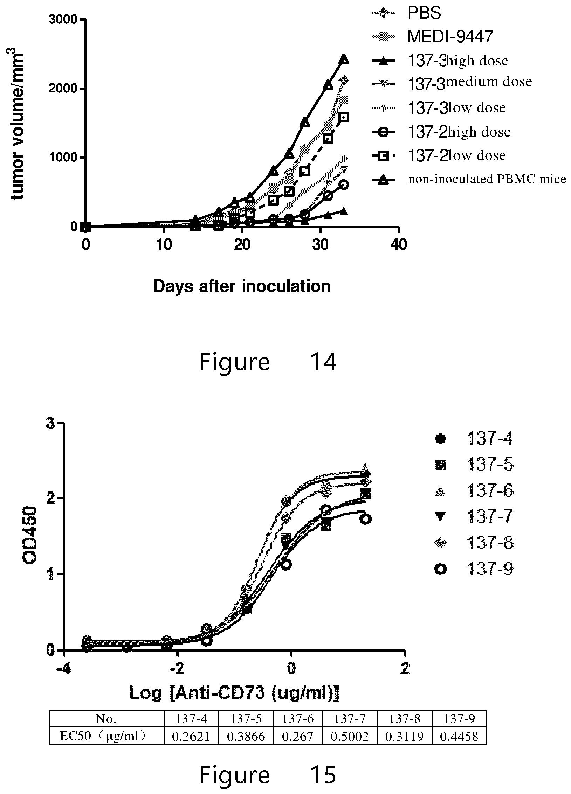

The results of binding of humanized antibodies 137-4, 137-5, 137-6, 137-7, 137-8, 137-9 antibodies to recombinant human CD73 are shown in Table 3 and FIG. 15.

**Table 3. Affinity results of humanized CD73 antibody and recombinant human CD73**

| No. | 137-4 | 137-5 | 137-6 | 137-7 | 137-8 | 137-9 |
|---|---|---|---|---|---|---|
| EC50 (µg/ml) | 0.2621 | 0.3866 | 0.267 | 0.5002 | 0.3119 | 0.4458 |

### (2) binding to recombinant monkey CD73 protein

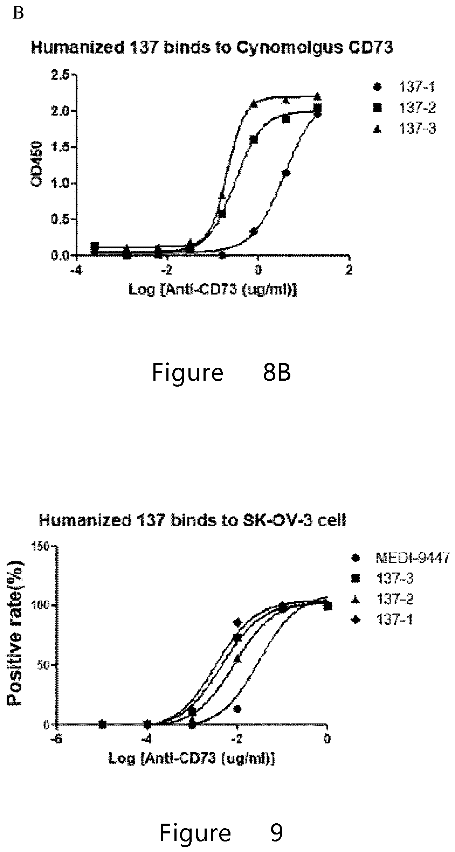

The recombinant monkey CD73 protein was packaged in plates according to a 3 µg/mL and a gradient diluted humanized CD73 antibodies (137-1, 137-2, 137-3) was added. The combination of antibody and the recombinant monkey CD73 was detected by an ELISA method, and the result is shown in FIG. 8B.

The results are shown in FIG. 8B, Table 4.

**Table 4. EC50 for binding of humanized CD73 antibody to recombinant monkey CD73 protein**

| Humanized CD73 antibody No. | 137-1 | 137-2 | 137-3 |
|---|---|---|---|
| EC50 (µg/ml) | 3.763 | 0.3132 | 0.2016 |

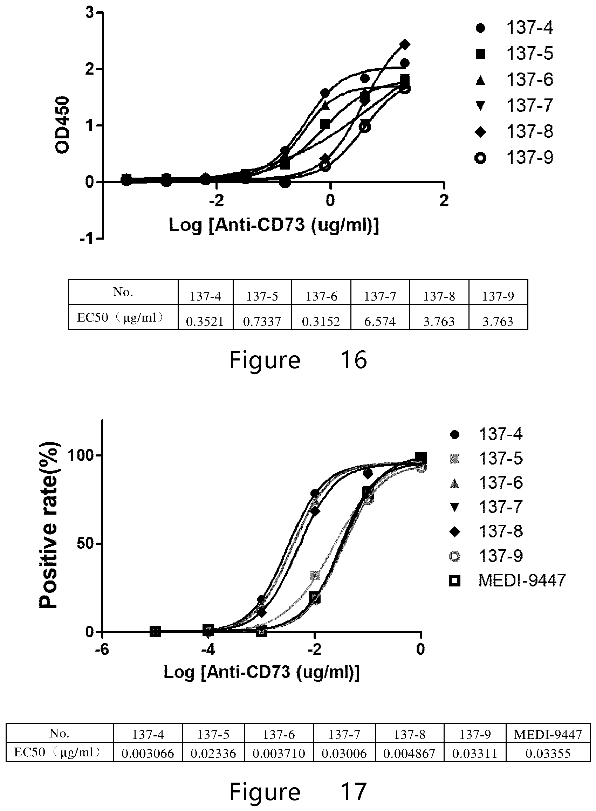

Binding of humanized antibodies 137-4, 137-5, 137-6, 137-7, 137-8, 137-9 antibodies to recombinant monkey CD73 protein are shown in Table 5 and FIG.16.

**Table 5. Affinity results of humanized CD73 antibody and recombinant monkey CD73**

| No. | 137-4 | 137-5 | 137-6 | 137-7 | 137-8 | 137-9 |
|---|---|---|---|---|---|---|
| EC50 (µg/ml) | 0.3521 | 0.7337 | 0.3152 | 6.574 | 3.763 | 3.763 |

### (3) Binding to human CD73 positive cells

4×10⁵ SK-OV-3 cells were taken, a gradient diluted humanized CD73 antibodies (137-1, 137-2, 137-3) was added, incubated for 1 h, washed three times with PBS, Anti-hFC-APC (purchased from Jackson immunology) was added, and detected by flow cytometry. S-curves were plotted and the results are shown in Figure 9.

The results are shown in Table 6 and Figure 9.

**Table 6. EC50 for binding of humanized CD73 antibody to SK-OV-3 cells**

| Antibody Name | 137-1 | 137-2 | 137-3 | MEDI-9477 |
|---|---|---|---|---|
| EC50 (µg/ml) | 2.135 | 3.264 | 7.026 | 23.486 |

Results show that the combination of humanized CD73 antibodies (137-1, 137-2, 137-3) and SK-OV-3 cells is superior to MEDI-9447.

The results of binding of humanized antibodies 137-4, 137-5, 137-6, 137-7, 137-8, 137-9 antibodies to SK-OV-3 cells are shown in Table 7 and FIG.17.

**Table 7. Affinity results of humanized CD73 antibody and SK-OV-3 cells**

| No. | 137-4 | 137-5 | 137-6 | 137-7 | 137-8 | 137-9 | MEDI -9447 |
|---|---|---|---|---|---|---|---|
| EC50 (µg/ml) | 0.003066 | 0.02336 | 0.003710 | 0.03006 | 0.004867 | 0.03311 | 0.03355 |

### (4) Binding to monkey CD73 positive cells

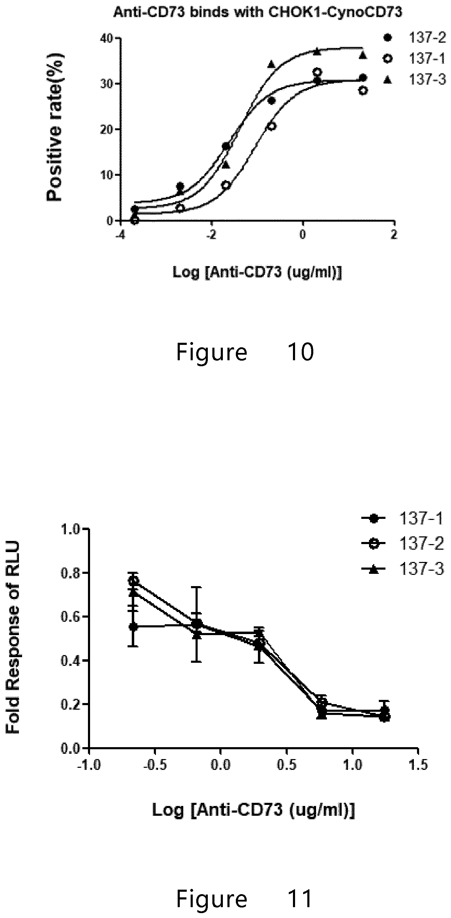

The full length of monkey CD73 was constructed on a eukaryotic expression vector containing NEO resistance, CHO-K1 cells were transfected, G418 was used for screening to obtain a CHOK1-cynoCD73 cell strain. 4×10⁵ CHOK1-cynoCD73 cells were taken and a gradient diluted humanized CD73 antibodies (137-1, 137-2, 137-3) were added, after incubation was performed for 1 h, the cells were washed with PBS for three times, Anti-Hail-APC (purchased from Jackson Immunology) was added, and detected by flow cytometry. S-curves were plotted and the results are shown in Figure 10.

The results are shown in Table 8.

**Table 8. EC50 for binding of humanized CD73 antibody to monkey CD73 positive cells**

| Humanized CD73 antibody No. | 137-1 | 137-2 | 137-3 |
|---|---|---|---|
| EC50 (µg/ml) | 0.08622 | 0.0233 | 0.04027 |

The results show that the humanized CD73 antibody has a very high binding activity with monkey CD73 positive cells.

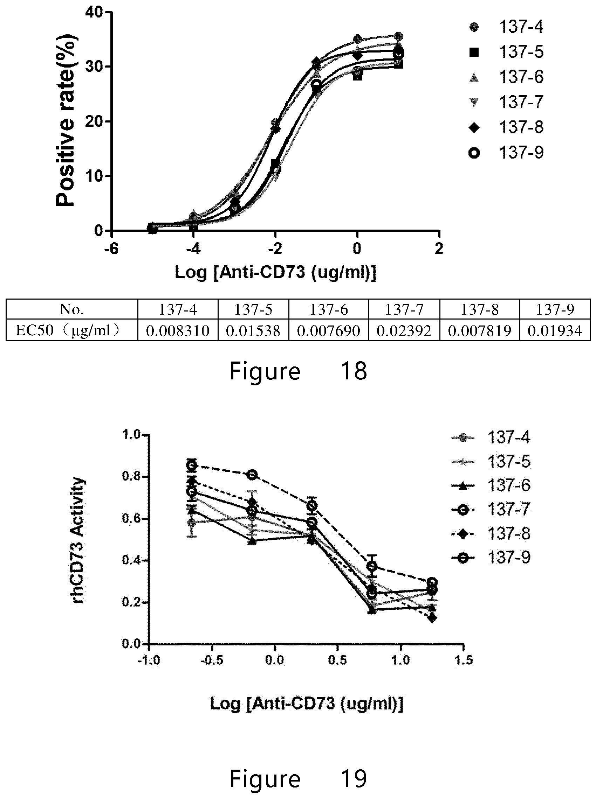

The results of binding of humanized antibodies 137-4, 137-5, 137-6, 137-7, 137-8, 137-9 antibodies to monkey CD73 positive cells are shown in Table 9 and FIG. 18.

**Table 9. Affinity results of humanized CD73 antibody and CHOK1-cynoCD73 cells**

| No. | 137-4 | 137-5 | 137-6 | 137-7 | 137-8 | 137-9 |
|---|---|---|---|---|---|---|
| EC50 (µg/ml) | 0.008310 | 0.01538 | 0.007690 | 0.02392 | 0.007819 | 0.01934 |

### Example 6 Inhibition Evaluation of CQ137 Humanized Enzyme Activity

In this embodiment, the humanized 137 antibody is mainly shown to inhibit CD73 protein and cell enzyme activity. Specifically,

### (1) CD73 protease activity inhibition

The CD73 protein was diluted to a working concentration of 5 µg/mL, a gradient diluted humanized CD73 antibodies (137-1, 137-2, 137-3) were added, incubation was performed at 37°C for 15 min, a mixed solution of 1nM ATP and AMP was added, incubation was performed at 37°C for 30 min, a CellTiter-GLo detection reagent (purchased from promega) was added in equal volumes, a self-luminescence value was read by an ELISA instrument, and the enzyme activity was calculated as is 1 without the addition of antibody, and the change in rhCD73 activity was obtained and shown in see FIG.11.

FIG. 19 shows that all the 6 humanized CD73 antibodies can inhibit the enzymatic activity of recombinant human CD73.

### (2) Inhibition of CD73 enzyme activity on human SK-OV-3 cells

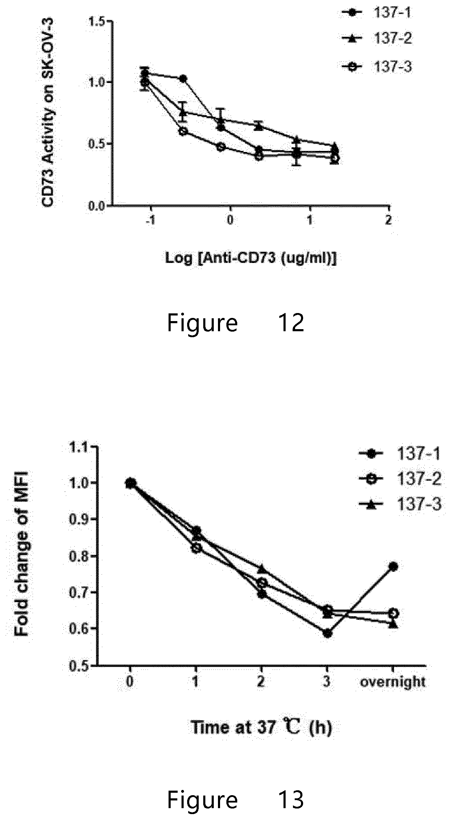

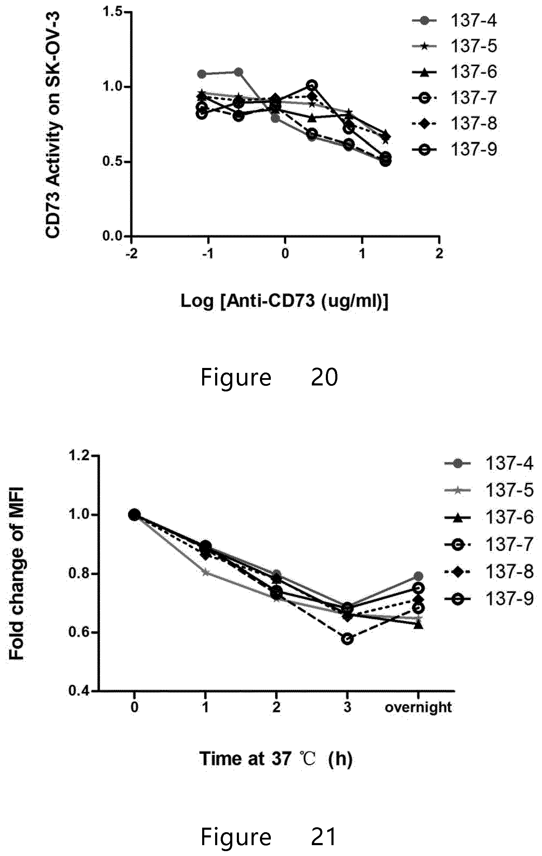

5×10⁴ SK-OV-3 cells were taken, a gradient diluted humanized CD73 antibodies (137-1, 137-2, 137-3, 137-4, 137-5, 137-6, 137-7, 137-8, 137-9) were added, incubated at 37°C for 15 min, 1nM AMP was added at 37°C for 2h, 1nM ATP was added, and a CellTiter-GLo detection reagent was added immediately, a self-luminescence value was read by an ELISA instrument, and the enzyme activity was calculated as 1 without the addition of antibody, and the change in rhCD73 activity was obtained, see FIG.12 and FIG.20.

The results show that the 9 humanized 137 antibodies can inhibit CD73 protein and CD73 enzyme activity on cell.

### Example 7 Humanized 137 Antibody-Mediated Cell Internalization

3×10⁵ SK-OV-3 cells were taken and incubated with 0.2µg CD73 antibody at 37°C. The cells were incubated for 0, 1, 2, 3 hours and overnight. After incubation, an equal amount of corresponding humanized CD73 antibodies (137-1,137-2,137-3, 137-4, 137-5, 137-6, 137-7, 137-8, 137-9) were added, after incubation for 1h, Anti-Hfc-APC flow antibody was added, and detected by flow cytometer after incubation and elution. The relative fluorescence intensity (MFI) at 0h is 1, and the calculated results are shown in FIG. 13 and FIG. 21.

Experimental results show that the 9 humanized antibodies of the present invention may also induce tumor cell internalization of CD73.

### Example 8 Humanized 137 Antibodies Inhibit Proliferation of Human Melanoma Cells A375 in Mice

Mouse model NSG-A375 of human melanoma cells (A375) was constructed by NSG mice, and inoculated human PBMC laboratory mice and non-inoculated human PBMC control mice were prepared, administered every other day, and tumor volume was measured. Experimental mice were set with solvent PBS administration blank control, MEDI-9447 low dose (low dose is 0.5 mpk) administration, 137-2 high dose (3 mpk) and low dose, 137-3 high, medium and low doses (medium dose is 1 mpk), and experimental results were shown in FIG. 14. The mpk (Milligrams Per Kilocalms) is mg/kg.

The results show that the anti-tumor effects of 137-2 and 137-3 at low doses are all significantly higher than that of MEDI-9447.

### Sequence information of the present invention

The CDRs for CQ137 and its humanized antibody are summarized in Table 10 below:

**Table 10**

| | CQ13 7 | 137-1 | 137-2 | 137-3 | 137-4 | 137-5 | 137-6 | 137-7 | 137-8 | 137-9 |
|---|---|---|---|---|---|---|---|---|---|---|
| VH CDR1 | 15 | 21 | 15 | 15 | 15 | 15 | 15 | 15 | 15 | 15 |
| VH CDR2 | 16 | 16 | 16 | 16 | 16 | 16 | 16 | 16 | 16 | 16 |
| VH CDR3 | 17 | 17 | 17 | 17 | 22 | 23 | 24 | 25 | 26 | 27 |
| VL CDR1 | 18 | 18 | 18 | 18 | 18 | 18 | 18 | 18 | 18 | 18 |
| VL CDR2 | 19 | 19 | 19 | 19 | 19 | 19 | 19 | 19 | 19 | 19 |
| VL CDR3 | 20 | 20 | 20 | 20 | 28 | 29 | 30 | 31 | 32 | 33 |

The sequence information of the present invention is shown in the following table:

| SEQ ID NO. | Name | Sequence |
|---|---|---|
| 1 | CQ137VL | DILMTQSPSSMYASLGERVTITCKASQDINTYLTWFQQ KPGKSPKTLIYRANILVDGVPSRFSGSGSGQDYSLTISS LEFEDMGIYYCLQYDEFPLTFGAGTKLELKR |
| 2 | CQ137VH | EVQLQQSGPELVKPGASVKISCKASGYSFTGYYIHWVK QSHVKSLEWIGRINPYNGATTYNQNFKDKASLTVDKS SSTAYMELHSLTSEDSAVYYCARFHYGAPDYWGQGTT LTVSS |
| 3 | CQ137-CH | |
| 4 | CQ137-CL | TVAAPSVFIFPPSDEQLKSGTASVVCLLNNFYPREAKV QWKVDNALQSGNSQESVTEQDSKDSTYSLSSTLTLSK ADYEKHKVYACEVTHQGLSSPVTKSFNRGEC |
| 5 | 137-1VH | QVQLVQSGAEVKKPGASVKVSCKASGYTFTGYYIHW VRQAPGQGLEWMGRINPYNGATTYNQNFKDRVTMTV DKSTSTAYMELRSLRSDDTAVYYCARFHYGAPDYWG QGTLVTVSS |
| 6 | 137-1VL | DIQMTQSPSSLSASVGDRVTITCKASQDINTYLTWFQQ KPGKAPKTLIYRANILVDGVPSRFSGSGSGQDYTLTISS LQPEDFATYYCLQYDEFPLTFGGGTKVEIKR |
| 7 | 137-2VH | QVQLVQSGAEVKKPGASVKVSCKASGYSFTGYYIHWV RQAPGQGLEWMGRINPYNGATTYNQNFKDRVTMTVD KSTSTAYMELRSLRSDDTAVYYCARFHYGAPDYWGQ GTLVTVSS |
| 8 | 137-2VL | DIQMTQSPSSLSASVGDRVTITCKASQDINTYLTWFQQ KPGKAPKSLIYRANILVDGVPSRFSGSGSGTDFTLTISSL QPEDFATYYCLQYDEFPLTFGGGTKVEIKR |
| 9 | 137-3VH | QVQLVQSGAEVKKPGSSVKVSCKASGYSFTGYYIHWV RQAPGQGLEWMGRINPYNGATTYNQNFKDRVTITVDK STSTAYMELSSLRSEDTAVYYCARFHYGAPDYWGQGT LVTVSS |
| 10 | 137-3VL | DIQMTQSPSSLSASVGDRVTITCKASQDINTYLTWYQQ |
| | | KPGKAPKTLIYRANILVDGVPSRFSGSGSGTDFTLTISSL QPEDFATYYCLQYDEFPLTFGGGTKVEIKR |
| 11 | CQ137VH | GAGGTTCAGCTGCAGCAGTCTGGACCTGAGCTGGTG AAGCCTGGGGCTTCAGTGAAGATATCCTGCAAGGCT TCTGGTTACTCATTCACTGGCTACTACATCCACTGGG TGAAGCAAAGCCATGTAAAGAGCCTTGAGTGGATTG GACGTATTAATCCTTACAATGGTGCTACTACCTACAA CCAGAATTTCAAGGACAAGGCCAGCTTGACTGTAGA TAAGTCCTCCAGCACAGCCTACATGGAGCTCCACAG CCTGACATCTGAGGACTCTGCAGTCTATTACTGTGCA AGATTCCACTACGGTGCCCCTGACTACTGGGGCCAA GGCACCACTCTCACAGTCTCCTCG |
| 12 | CQ137VL | GATATTCTGATGACCCAATCTCCATCTTCCATGTATG CATCTCTAGGAGAGAGAGTCACTATCACTTGCAAGG CGAGTCAGGACATTAATACCTATTTAACTTGGTTCCA GCAGAAACCAGGGAAATCTCCTAAGACCCTGATCTA TCGTGCAAACATATTGGTAGATGGGGTCCCATCAAG GTTCAGTGGCAGTGGATCTGGGCAAGATTATTCTCTC ACCATCAGCAGCCTGGAGTTTGAAGATATGGGAATT TATTATTGTCTACAGTATGATGAGTTTCCGCTCACGT TCGGTGCTGGGACCAAGCTGGAGCTGAAACGT |
| 13 | CQ137-CH | GCCTCCACCAAGGGCCCATCGGTCTTCCCCCTGGCA CCCTCCTCCAAGAGCACCTCTGGGGGCACAGCGGCC CTGGGCTGCCTGGTCAAGGACTACTTCCCCGAACCG GTGACGGTGTCGTGGAACTCAGGCGCCCTGACCAGC GGCGTGCACACCTTCCCGGCTGTCCTACAGTCCTCAG GACTCTACTCCCTCAGCAGCGTGGTGACCGTGCCCTC CAGCAGCTTGGGCACCCAGACCTACATCTGCAACGT GAATCACAAGCCCAGCAACACCAAGGTGGACAAGA AAGTTGAGCCCAAATCTTGTGACAAAACTCACACAT GCCCACCGTGCCCAGCACCTGAACTCCTGGGGGGAC CGTCAGTCTTCCTCTTCCCCCCAAAACCCAAGGACAC CCTCATGATCTCCCGGACCCCTGAGGTCACATGCGT GGTGGTGGACGTGAGCCACGAAGACCCTGAGGTCAA GTTCAACTGGTACGTGGACGGCGTGGAGGTGCATAA TGCCAAGACAAAGCCGCGGGAGGAGCAGTACAACA GCACGTACCGGGTGGTCAGCGTCCTCACCGTCCTGC ACCAGGACTGGCTGAATGGCAAGGAGTACAAGTGC AAGGTCTCCAACAAAGCCCTCCCAGCCCCCATCGAG AAAACCATCTCCAAAGCCAAAGGGCAGCCCCGAGA ACCACAGGTGTACACCCTGCCCCCATCCCGGGATGA GCTGACCAAGAACCAGGTCAGCCTGACCTGCCTGGT CAAAGGCTTCTATCCCAGCGACATCGCCGTGGAGTG GGAGAGCAATGGGCAGCCGGAGAACAACTACAAGA CCACGCCTCCCGTGCTGGACTCCGACGGCTCCTTCTT CCTCTACAGCAAGCTCACCGTGGACAAGAGCAGGTG GCAGCAGGGGAACGTCTTCTCATGCTCCGTGATGCA |
| | | TGAGGCTCTGCACAACCACTACACGCAGAAGAGCCT CTCCCTGTCTCCGGGTAAATGA |
| 14 | CQ137-CL | ACGGTGGCTGCACCATCTGTCTTCATCTTCCCGCCAT CTGATGAGCAGTTGAAATCTGGAACTGCCTCTGTTGT GTGCCTGCTGAATAACTTCTATCCCAGAGAGGCCAA AGTACAGTGGAAGGTGGATAACGCCCTCCAATCGGG TAACTCCCAGGAGAGTGTCACAGAGCAGGACAGCA AGGACAGCACCTACAGCCTCAGCAGCACCCTGACGC TGAGCAAAGCAGACTACGAGAAACACAAAGTCTAC GCCTGCGAAGTCACCCATCAGGGCCTGAGCTCGCCC GTCACAAAGAGCTTCAACAGGGGAGAGTGTTAG |
| 15 | VH-CDR1 | GYSFTGYY |
| 16 | VH-CDR2 | INPYNGAT |
| 17 | VH-CDR3 | ARFHYGAPDY |
| 18 | VL-CDR1 | QDINTY |
| 19 | VL-CDR2 | RAN |
| 20 | VL-CDR3 | LQYDEFPLT |
| 21 | 137-1VH-C DR1 | GYTFTGYY |
| 22 | 137-4VH-C DR3 | ARFHYGAVDY |
| 23 | 137-5VH-C DR3 | ARFHYGAADY |
| 24 | 137-6VH-C DR3 | ARFHYGAPEY |
| 25 | 137-7VH-C DR3 | ARFHYGAPNY |
| 26 | 137-8VH-C DR3 | ARFHYGAPDF |
| 27 | 137-9VH-C DR3 | ARFHYGAPDL |
| 28 | 137-4VL-C DR3 | LQYEEFPLT |
| 29 | 137-5VL-C DR3 | LQYNEFPLT |
| 30 | 137-6VL-C DR3 | LQYAEFPLT |
| 31 | 137-7VL-C DR3 | LQYDDFPLT |
| 32 | 137-8VL-C DR3 | LQYDQFPLT |
| 33 | 137-9VL-C DR3 | LQYDAFPLT |
| 34 | 137-4 VH | QVQLVQSGAEVKKPGSSVKVSCKASGYSFTGYYIHWV RQAPGQGLEWMGRINPYNGATTYNQNFKDRVTITVDK STSTAYMELSSLRSEDTAVYYCARFHYGAVDYWGQG TLVTVSS |
| 35 | 137-5 VH | QVQLVQSGAEVKKPGSSVKVSCKASGYSFTGYYIHWV RQAPGQGLEWMGRINPYNGATTYNQNFKDRVTITVDK STSTAYMELSSLRSEDTAVYYCARFHYGAADYWGQG TLVTVSS |
| 36 | 137-6 VH | QVQLVQSGAEVKKPGSSVKVSCKASGYSFTGYYIHWV RQAPGQGLEWMGRINPYNGATTYNQNFKDRVTITVDK STSTAYMELSSLRSEDTAVYYCARFHYGAPEYWGQGT LVTVSS |
| 37 | 137-7 VH | QVQLVQSGAEVKKPGSSVKVSCKASGYSFTGYYIHWV RQAPGQGLEWMGRINPYNGATTYNQNFKDRVTITVDK STSTAYMELSSLRSEDTAVYYCARFHYGAPNYWGQGT LVTVSS |
| 38 | 137-8 VH | QVQLVQSGAEVKKPGSSVKVSCKASGYSFTGYYIHWV RQAPGQGLEWMGRINPYNGATTYNQNFKDRVTITVDK STSTAYMELSSLRSEDTAVYYCARFHYGAPDFWGQGT LVTVSS |
| 39 | 137-9 VH | QVQLVQSGAEVKKPGSSVKVSCKASGYSFTGYYIHWV RQAPGQGLEWMGRINPYNGATTYNQNFKDRVTITVDK STSTAYMELSSLRSEDTAVYYCARFHYGAPDLWGQGT LVTVSS |
| 40 | 137-4 VL | DIQMTQSPSSLSASVGDRVTITCKASQDINTYLTWYQQ KPGKAPKTLIYRANILVDGVPSRFSGSGSGTDFTLTISSL QPEDFATYYCLQYEEFPLTFGGGTKVEIKR |
| 41 | 137-5 VL | DIQMTQSPSSLSASVGDRVTITCKASQDINTYLTWYQQ KPGKAPKTLIYRANILVDGVPSRFSGSGSGTDFTLTISSL QPEDFATYYCLQYNEFPLTFGGGTKVEIKR |
| 42 | 137-6 VL | DIQMTQSPSSLSASVGDRVTITCKASQDINTYLTWYQQ KPGKAPKTLIYRANILVDGVPSRFSGSGSGTDFTLTISSL QPEDFATYYCLQYAEFPLTFGGGTKVEIKR |
| 43 | 137-7 VL | DIQMTQSPSSLSASVGDRVTITCKASQDINTYLTWYQQ KPGKAPKTLIYRANILVDGVPSRFSGSGSGTDFTLTISSL QPEDFATYYCLQYDDFPLTFGGGTKVEIKR |
| 44 | 137-8 VL | DIQMTQSPSSLSASVGDRVTITCKASQDINTYLTWYQQ KPGKAPKTLIYRANILVDGVPSRFSGSGSGTDFTLTISSL QPEDFATYYCLQYDQFPLTFGGGTKVEIKR |
| 45 | 137-9 VL | DIQMTQSPSSLSASVGDRVTITCKASQDINTYLTWYQQ KPGKAPKTLIYRANILVDGVPSRFSGSGSGTDFTLTISSL QPEDFATYYCLQYDAFPLTFGGGTKVEIKR |
| 46 | hIgG1 constant region | |
| | | |
| 47 | Kappa chain constant region | TVAAPSVFIFPPSDEQLKSGTASVVCLLNNFYPREAKV QWKVDNALQSGNSQESVTEQDSKDSTYSLSSTLTLSK ADYEKHKVYACEVTHQGLSSPVTKSFNRGEC |

| | | |
|---|---|---|
| Note: VL is a light chain variable region, VH is a heavy chain variable region, CH is a heavy chain constant region, CL is a light chain constant region, VH-CDR1, VH-CDR2, VH-CDR3 are CDR1, CDR2, CDR3 of the heavy chain variable region; VL-CDR1, VL-CDR2, VL-CDR3 are CDR1, CDR2, CDR3 of the light chain variable region. | | |

All literatures mentioned in the present application are incorporated herein by reference, as though each one is individually incorporated by reference. In addition, it should also be understood that, after reading the above teachings of the present invention, those skilled in the art can make various changes or modifications, equivalents of which falls in the scope of claims as defined in the appended claims.

## Claims

1. An anti-human CD73 antibody, wherein the antibody comprises a light chain and a heavy chain,
and the light chain variable region of the light chain comprises the following three light chain CDRs:
VL-CDR1 as shown in SEQ ID NO.: 18,
VL-CDR2 as shown in SEQ ID NO.: 19, and
VL-CDR3 as shown in SEQ ID NO.:20, 28, 29, 30, 31, 32 or 33;
wherein, the heavy chain variable region of the heavy chain comprises the following three heavy chain CDRs:
VH-CDR1 as shown in SEQ ID NO.: 15 or 21,
VH-CDR2 as shown in SEQ ID NO.: 16, and
VH-CDR3 as shown in SEQ ID NO.:17, 22, 23, 24, 25, 26 or 27.

2. The antibody of claim 1, wherein the light chain of the antibody comprises the three light chain CDRs and a light chain framework region for connecting the light chain CDR; and the heavy chain of the antibody comprises the three heavy chain CDRs and a heavy chain framework region for connecting the heavy chain CDR.

3. A recombinant protein, wherein the recombinant protein has:
(i) a light chain and/or a heavy chain, or an anti-CD73 antibody formed by the light chain and the heavy chain,
wherein, the light chain variable region of the light chain comprises
VL-CDR1 as shown in SEQ ID NO.: 18,
VL-CDR2 as shown in SEQ ID NO.: 19, and
VL-CDR3 as shown in SEQ ID NO.:20, 28, 29, 30, 31, 32 or 33;
and the heavy chain variable region of the heavy chain comprises
VH-CDR1 as shown in SEQ ID NO.: 15 or 21,
VH-CDR2 as shown in SEQ ID NO.: 16, and
VH-CDR3 as shown in SEQ ID NO.:17, 22, 23, 24, 25, 26 or 27;
and (ii) optionally a tag sequence assisting expression and/or purification.

4. An antibody preparation, wherein the antibody preparation comprises:
(a) the antibody of claim 1; and
(b) a carrier, wherein the carrier comprises: a buffering agent, a sterile water, and an optional surfactant.

5. A kit, wherein the kit contains the antibody preparation of claim 4, and a container containing the antibody preparation.

6. A CAR construct, wherein the scFv segment of the antigen-binding region of the CAR construct is a binding region that specifically binds to CD73 and the scFv segment has a light chain variable region and a heavy chain variable region, wherein the light variable region comprises
VL-CDR1 as shown in SEQ ID NO.: 18,
VL-CDR2 as shown in SEQ ID NO.: 19, and
VL-CDR3 as shown in SEQ ID NO.:20, 28, 29, 30, 31, 32 or 33;
and the heavy chain variable region comprises
VH-CDR1 as shown in SEQ ID NO.: 15 or 21,
VH-CDR2 as shown in SEQ ID NO.: 16, and
VH-CDR3 as shown in SEQ ID NO.:17, 22, 23, 24, 25, 26 or 27.

7. A recombinant immune cell, wherein the immune cell expresses an exogenous CAR construct of claim 6.

8. An antibody drug conjugate comprising:
(a) an antibody moiety selected from the group consisting of the antibody of claim 1, the recombinant protein of claim 3, and a combination thereof; and
(b) a coupling moiety coupled to the antibody moiety, and the coupling moiety is selected from the group consisting of a detectable label, a drug, a toxin, a cytokine, a radionuclide, an enzyme, or a combination thereof.

9. Use of an active ingredient, wherein the active ingredient is selected from the group consisting of the antibody of claim 1, the recombinant protein of claim 3, the CAR construct of claim 6, the immune cell of claim 7, the antibody drug conjugate of claim 8, and a combination thereof, wherein the active ingredient is used for:
(a) preparing a detection reagent or a kit;
(b) preparing a drug or preparation for preventing and/or treating CD73-related diseases; and/or
(c) preparing a drug or preparation for preventing and/or treating cancers or tumors associated with CD73-related diseases.

10. A pharmaceutical composition containing:
(i) an active ingredient, which is selected from the group consisting of: the antibody of claims 1, the recombinant protein of claim 3, or the CAR construct of claim 6, the immune cell of claim 7, the antibody drug conjugate of claim 8, and a combination thereof; and
(ii) a pharmaceutically acceptable carrier.

11. A polynucleotide, wherein the polynucleotide encodes a polypeptide selected from group consisting of:
(1) the antibody of claim 1; or
(2) the recombinant protein of claim 3.
(3) the CAR construct of claim 6.

12. A vector comprising the polynucleotide of claim 11.

13. A genetically engineered host cell, wherein the host cell comprises the vector of claim 12, or the genome thereof is integrated with the polynucleotide of claim 11.

14. A method for *in vitro* non-diagnostic detection of CD73 protein in a sample, wherein the method comprises the steps:
(1) contacting the sample with the antibody of claims 1 *in vitro*;
(2) detecting the formation of an antigen-antibody complex, wherein the formation of a complex indicates the presence of CD73 protein in the sample.
